# EUROPEAN PATENT SPECIFICATION

(11) **EP 1 699 289 B1**
(45) Date of publication and mention of the grant of the patent: **26.11.2008**
(21) Application number: 04808891.8
(22) Date of filing: 22.12.2004
(51) Int. Cl.: A01K 67/027

(54) **NON-HUMAN MAMMAL COMPRISING A MODIFIED SERCA2 GENE AND METHODS, CELLS, GENES, AND VECTORS THEREOF**
NICHTMENSCHLICHES SÄUGETIER MIT MODIFIZIERTEM SERCA2-GEN SOWIE VERFAHREN, ZELLEN, GENE UND VEKTOREN HIERFÜR
MAMMIFERE NON HUMAIN COMPRENANT UN GENE SERCA2 MODIFIE ET SES METHODES, CELLULES, GENES ET VECTEURS

(30) Priority: 30.12.2003 US 533740 P
(43) Date of publication of application: 13.09.2006
(73) Proprietor: Ullevål Universitetssykehus HF, 0407 Oslo (NO)
(72) Inventor: CHRISTENSEN, Geir, N-0379 Oslo (NO); ANDERSSON, Kristin Brevik, N-1482 Nittedal (NO)
(74) Representative: Agvald-Glas, Kerstin Gunilla
(86) International application number: PCT/NO2004/000397
(87) International publication number: WO 2005/063007

(56) References cited:
- JI Y. ET AL: 'Disruption of a Single Copy of the SERCA2 Gene Results in Altered Ca2+ Homeostasis and Cardiomyocyte Function' THE JOURNAL OF BIOLOGICAL CHEMISTRY vol. 275, no. 48, 01 December 2000, pages 38073 - 38080, XP002986180
- SOHAL D.S. ET AL: 'Temporally Regulated and Tissue-Specific Gene Manipulations in the Adult and Embryonic Heart Using a Tamoxifen-Inducible Cre Protein' CIRCULATION RESEARCH vol. 89, 06 July 2001, pages 20 - 25, XP002986181
- VER HEYEN M. ET AL: 'Replacement of the Muscle-Specific Sarcoplasmic Reticulum Ca2+-ATPase Isoform SERCA2a by the Nonmuscle SERCA2b Homologue Causes Mild Concentric Hypertrophy and Impairs Contraction-Relaxation of the Heart' CIRCULATION RESEARCH vol. 89, 26 October 2001, pages 838 - 846, XP002986182
- PERIASAMY M. ET AL: 'Impaired Cardiac Performance in Heterozygous Mice with a Null Mutation in the Sarco(endo)plasmic Reticulum Ca2+-ATPase Isoform 2 (SERCA2) Gene' THE JOURNAL OF BIOLOGICAL CHEMISTRY vol. 274, no. 4, 22 January 1999, pages 2556 - 2562, XP002986183
- GAUSSIN V. ET AL: 'Endocardial cushion and myocardial defects after cardiac myocyte-specific conditional deletion of the bone morphogenetic protein receptor ALK3' PNAS vol. 99, no. 5, 05 March 2002, pages 2878 - 2883, XP002986184
- ROSSANT J. ET AL: '"Cre"-ating mouse mutants - a meeting review on conditional mouse genetics' GENES & DEVELOPMENT vol. 13, 1999, pages 142 - 145, XP001004604

## Description

### FIELD OF THE INVENTION

The present invention relates to a genetically modified mouse having its genomic sarco(endo)plasmic reticulum Ca²⁺ ATPase (Serca ATPase) gene modified. The invention also relates to a modified Serca ATPase gene, as well as eukaryotic cells and vectors containing this gene. Further, the invention relates to a method for screening a compound or a mixture of compounds for activity against defective Ca²⁺ handling. One purpose of the invention is to provide a tool to genetically affect Ca²⁺ handling in live mice in an organ of interest; e.g. in heart muscle to produce heart failure. The invention is particularly useful for studying heart failure and as a tool for developing novel drugs and methods for treatment of heart failure.

### BACKGROUND OF THE INVENTION

### Heart failure

Heart failure is defined as a pathophysiological state in which the heart is unable to pump blood to meet the body's metabolic demand. Heart failure has a high morbidity and mortality (2-5 years) after diagnosis. The number of heart failure patients has doubled the last 12 years in Western society, and results in a heavy load on the healthcare system. The most common causes are high blood pressure, repeated episodes of ischemia or myocardial infarction. Especially for the latter patient group, one expects an increase in heart failure at a later stage because of the increase in acute infarction survival rate.

### Post-infarction heart failure.

After an infarction, the mechanical load on the remaining non-infarcted cardiac tissue increases. A hypertrophic process is induced in the cardiomyocytes (increase in cell mass and contractile proteins) and a restructuring of the extracellular matrix. These processes are often termed remodeling. With time, the heart is not able to compensate for the strain, and progressive failure develops. It is unclear whether the hypertrophic process itself is an integral part of failure progression, or whether hypertrophy/remodeling and failure are two parallel processes. In general, the molecular mechanisms driving heart failure progression are not sufficiently understood, and the treatment regimens (beta-blockers, angiotensin converting enzyme (ACE) inhibitors) are mainly directed towards alleviating symptoms - not the underlying mechanistic problems. Thus, new animal models that realistically mimic heart failure in humans, combined with a better understanding of the molecular mechanisms, may give new avenues for the development of therapeutic strategies.

### Contractile function and molecular biology of heart failure

The three major determinants of contractile function in cardiac muscle cells are the trigger of Ca²⁺ release, reuptake of Ca²⁺ into the sarcoplasmic reticulum (SR) by Serca ATPases, and available SR Ca²⁺ content. Compared to normal cardiac tissue, the hallmark functional changes in post-infarction heart failure are an increase in the muscle relaxation time after contraction, reduced muscle fractional shortening, and reduced contractile force in the cardiomyocytes (Holt, Tønnessen et al. 1998; Hasenfuss and Pieske 2002). At the molecular level it is well documented that the efficiency of pumping Ca²⁺ back into the SR via the Serca ATPases, in particular the Serca2 ATPase, is strongly reduced (Hasenfuss and Pieske 2002; Sande, Sjaastad et al. 2002; Frank, Bolck et al. 2003).

Serca ATPases are major ATP-driven Ca²⁺ handling ion pumps in the cell. "Ca²⁺ handling" refers herein to the transportation of Ca²⁺ inside the cell, in and out of organelles, and in and out of the cell. Serca proteins exist as several protein isoforms, coded by the three genes Serca1, Serca2 and Serca3 (Loukianov, Ji et al. 1998; Wuytack, Raeymaekers et al. 2002, and references therein) Serca1 is predominant in fast-twitch skeletal muscle, Serca3 is expressed mainly in secretory tissues and in organs such as kidney and pancreas, whereas Serca2 is expressed in all tissues in the body. The Serca2a isoform is expressed in cardiomyocytes and slow-twitch skeletal muscle (very low levels in other tissues). In cardiac and slow-twitch skeletal muscle, Serca2 plays an essential role in muscle contraction. The Serca2b isoform is expressed in most cell types, including vascular smooth muscle, CNS, and non-neuronal tissue. Recently, a new isoform expressed in monocytes, Serca2c, was described in humans (Gelebart, Martin et al. 2003). Muscle pathology has been associated with mutations in the Serca class genes in humans (Loukianov, Ji et al. 1998; Shull, Okunade et al. 2003). Deletion of Serca1 and Serca2 genes in mice is neoneatal and early embryonic lethal, respectively (Periasamy, Reed et al. 1999; Pan, Zvaritch et al. 2003). In contrast, Serca3 null mice are viable, but display other defects (Arredouani, Guiot et al. 2002).

A number of studies have shown that Serca2 activity, Serca2 mRNA and protein content in failing hearts of both humans and experimental animals are reduced compared with non-failing hearts. It has been clearly demonstrated that increasing Serca2 expression level, either by adenoviral infection or in transgenic mice, improved both contractility and SR function in experimental animals (Periasamy and Huke 2001; Nakayama, Otsu et al. 2003). It has also been demonstrated that Serca1 is able to partially substitute for Serca2 function in the heart (Loukianov, Ji et al. 1998).

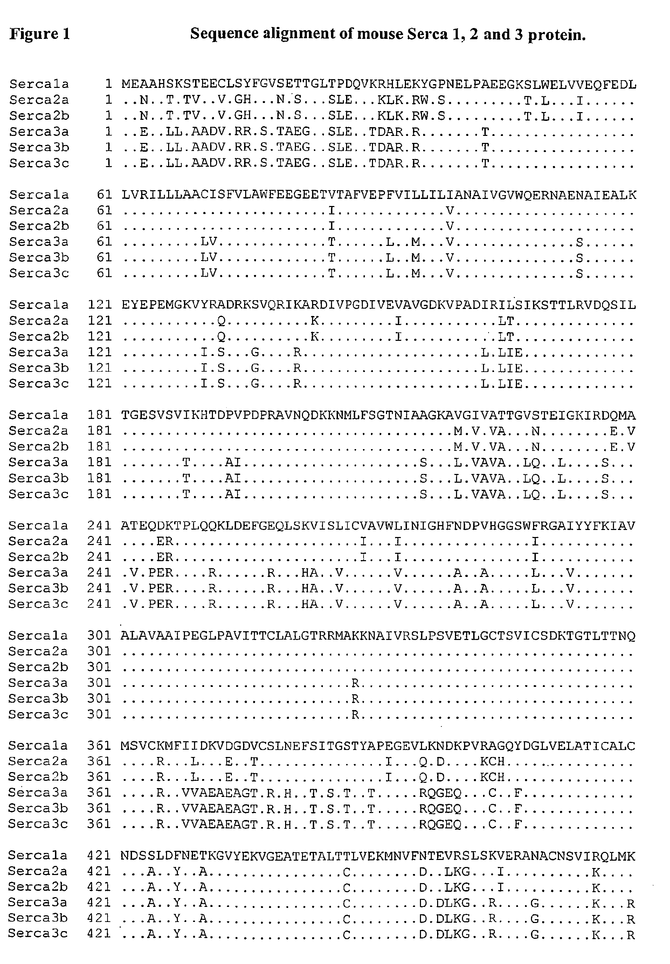

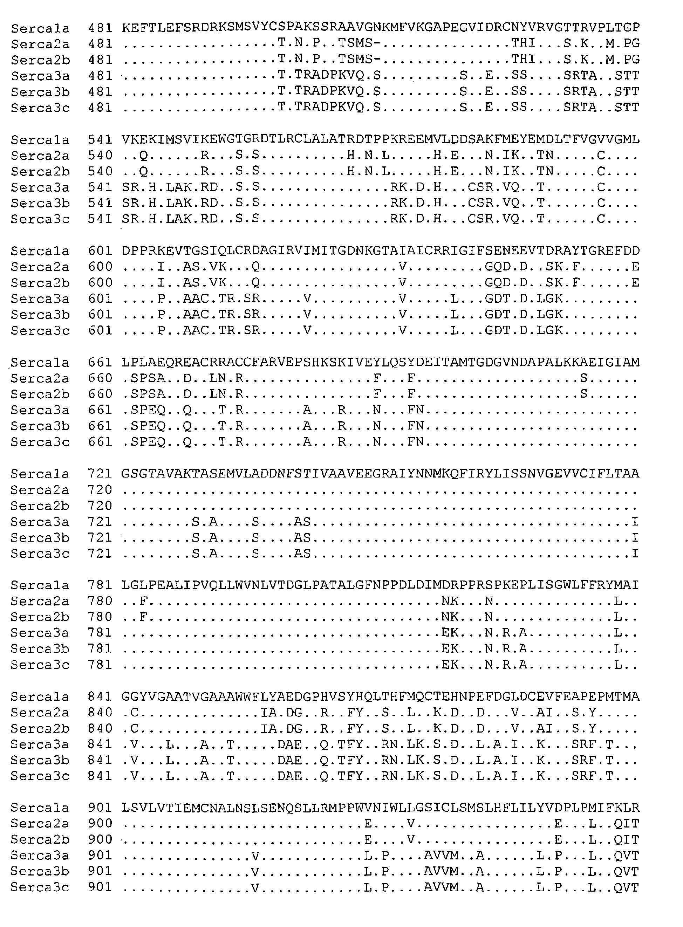

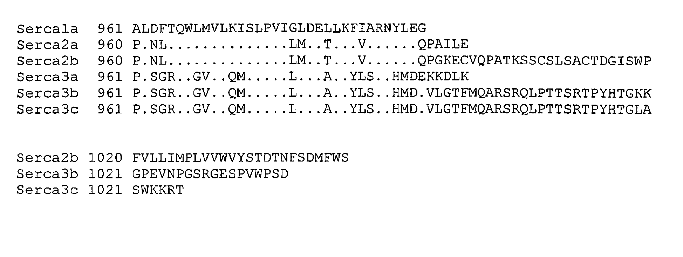

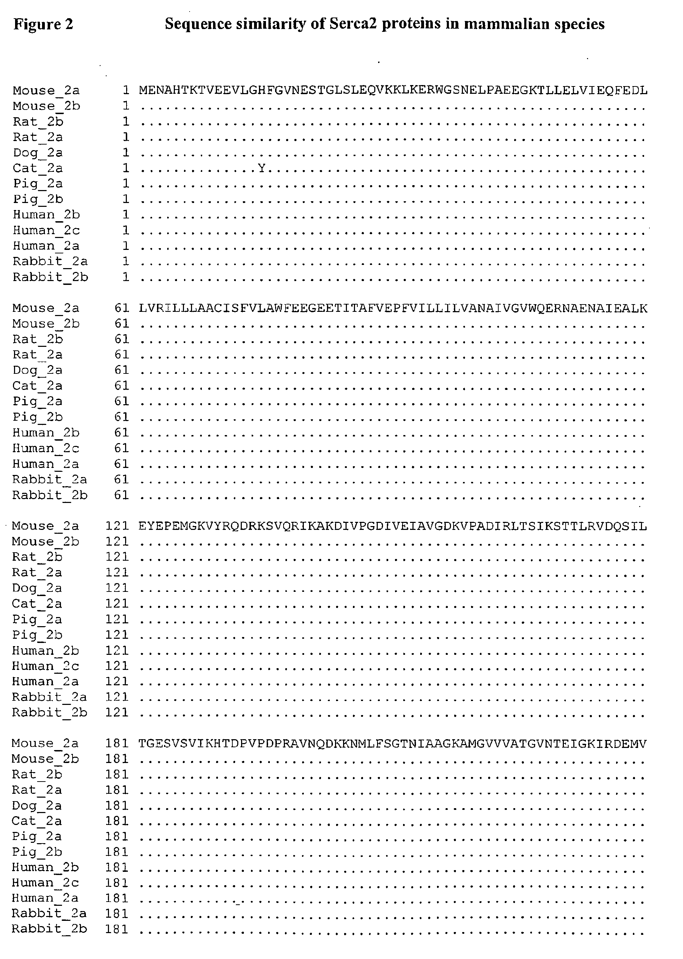

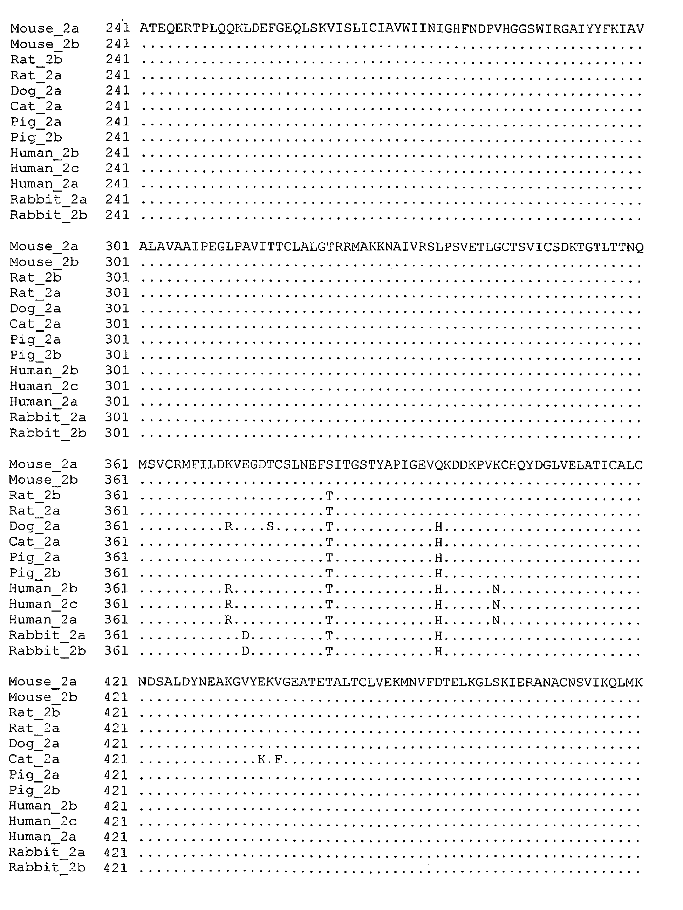

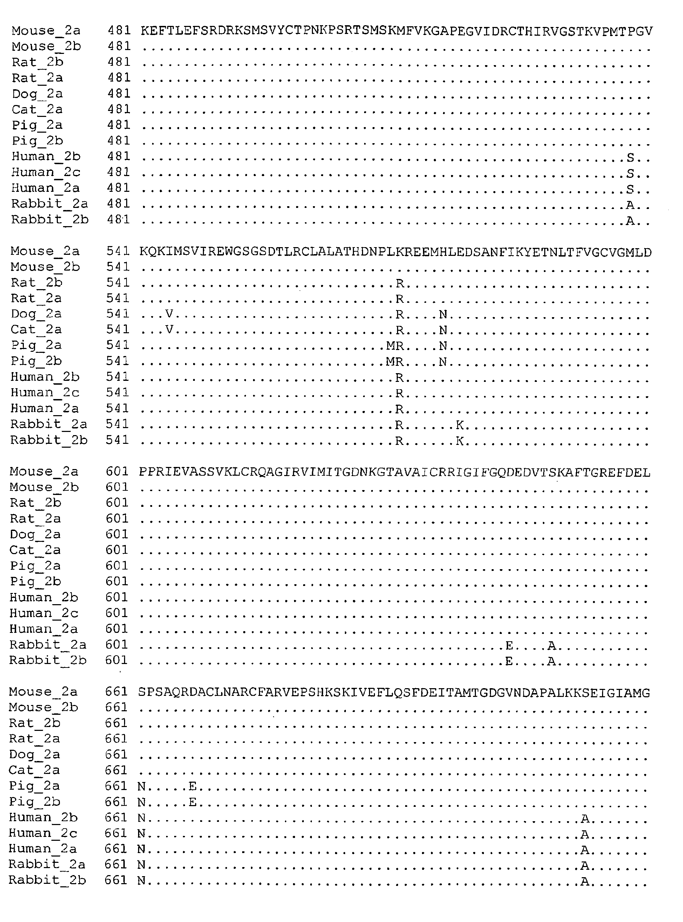

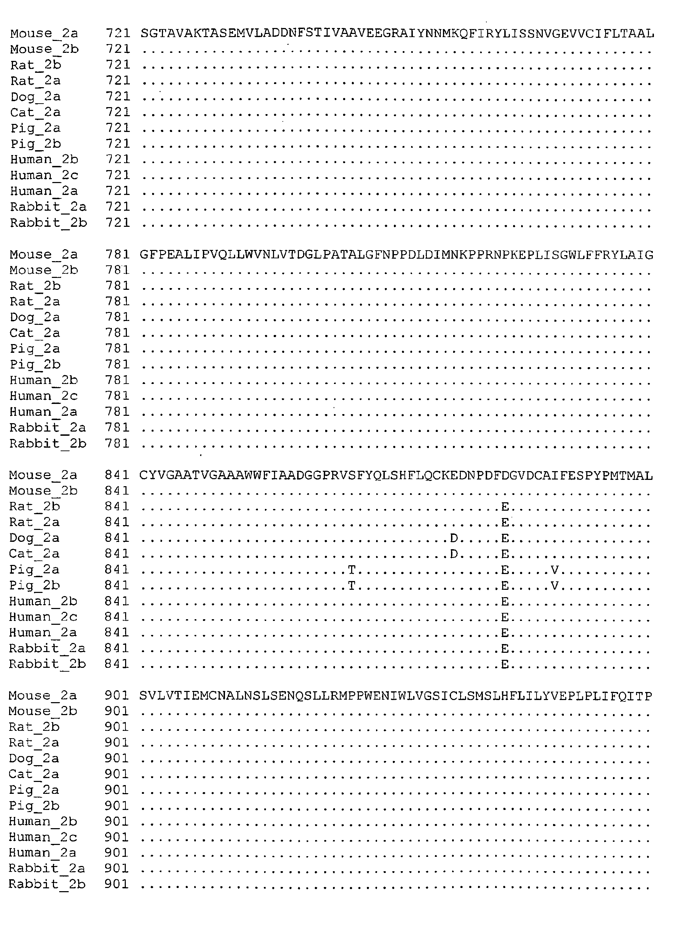

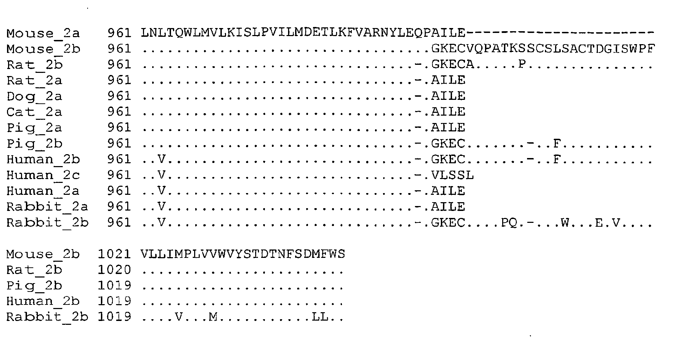

The amino acid sequence similarity between the Serca1, 2 and 3 protein isoforms within one species is very high (>98%), as exemplified by the alignment of murine Serca proteins isoforms shown in figure 1. Furthermore, all the Serca proteins are highly conserved in mammals. This high level of conservation is exemplified by the amino acid alignment of Serca2 from a range of mammalian species provided in figure 2.

### Serca and models of heart failure

The major disadvantage of traditional animal models where heart failure is induced by operative procedures is the difficulty of standardization, their unintended effects on the animals, as well as the difficulty of large-scale experiments required for therapeutic drug development. Another major obstacle in studies on heart failure is the lack of methods to distinguish between primary causes of defective Ca²⁺-handling and secondary adaptive processes. No adult animal models with defective Ca²⁺-handling to simulate heart failure have been provided to date and, as a consequence, drug screening and testing is rendered inefficient.

Given the role of Serca, particularly of Serca2, in heart failure and its high level of conservation throughout evolution, one approach to this problem is manipulation of the Serca2 gene in experimental animals. Two groups have previously developed mouse models with reduced Serca2 expression (Ji, Loukianov et al. 1999; Periasamy, Reed et al. 1999; Ver Heyen, Heymans et al. 2001; Antoons, Ver Heyen et al. 2003; Shull, Okunade et al. 2003). While a null mutation in the Serca2 gene (*atp2a2*) in mice is embryonic lethal, Serca2^{wt/null} heterozygous mice have a reduction in Serca2 protein and decreased contractility in the heart (Periasamy, Reed et al. 1999; Ji, Lalli et al. 2000; Huke, Liu et al. 2003). In a Serca2 isoform-specific mutant mouse model, Serca^{2b/b} (Serca2a^{null}) animals develop cardiac hypertrophy (Ver Heyen, Heymans et al. 2001). Both models have a 30-40% reduction in Serca2 ATPase protein and activity. Nevertheless, none of these mice develop heart failure. Differential changes in the expression of other proteins in these two models suggest different mechanisms of compensation for reduced Serca2 activity in these two models (Ji, Loukianov et al. 1999; Periasamy, Reed et al. 1999; Ver Heyen, Heymans et al. 2001; Antoons, Ver Heyen et al. 2003; Huke, Liu et al. 2003; Shull, Okunade et al. 2003). The fact that one allele of the Serca2 gene is inactivated at an early embryonic stage provides ample time to activate compensatory mechanisms. Hence, the above animal models are inadequate for heart failure research and drug development as heterozygous Serca2 mutants do not develop heart failure because they build up compensatory mechanisms from embryonic age, whereas homozygous Serca2 deletion mutants die in early embryonic life.

The present invention overcomes the above obstacles by providing a transgene mouse, in which the level of Serca ATPase function, more particular Serca2 ATPase, can be directly manipulated in adult animals in order to simulate defect Ca²⁺-handling in general, and heart failure in particular. This effect is obtained by employing inducible recombination systems, like for example Cre-loxP. Such systems allow both spatial (a given cell type) and temporal (a given time) control of mutations in living animals.

The system of Cre-loxP animals is based on two separate animal lines carrying two genetic components (described in U.S patent number 4,959,317). One animal line carries the gene of interest marked by loxP recombination sites (floxed gene). These sites are placed such that the gene of interest is inactivated when recombination takes place between the two recombination sites. This specific recombination reaction is catalyzed by the Cre recombinase. The second animal line expresses the Cre recombinase and directs the timing of gene destruction in the tissue or organ of interest.

When these two animal lines are, for example, bred together, the gene of interest is excised in the tissue or organ of interest. A similar recombination system for mammals has been developed, based on the frt recombination site and FLP recombinase for P1 bacteriophage.

The present invention is a valuable tool for studying medical conditions caused by defect Ca²⁺ handling, especially heart failure, as well as screening for, and further testing of, substances antagonizing such conditions.

### DESCRIPTION OF TABLES AND FIGURES

**Table 1 Genotype distribution of breeding results for Serca2^{flox} and MLC-2*v*-Cre knock-in mice.**
**Table 2 Body and organ weights for Serca2 cardiac heterozygous and control mice.** Age, body, heart and lung weights of control and heterozygous animals (Mean ± SEM).
**Table 3 Heart dimensions and flow parameters.** FF, Serca2^{flox/flox}; FC, Serca2^{wt/flox} MLC-2v^{wt/cre}; IVSd/s, inter-ventricular septum thickness in diastole respectively systole; LVDd/s, left ventricular diameter in diastole respectively systole; FS, fractional shortening in LVD; PWd/s, posterior wall thickness in diastole respectively systole; LAD, left atrial diameter; PWSV, posterior wall shortening velocity; PWRV, posterior wall relaxation velocity; LVOT, left ventricular outlet tract; RVOT, right ventricular outlet tract; CO, cardiac output; VTI, velocity time integral. All values are mean ± SEM
**Table 4 Haemodynamic pressure measurements.**
**Table 5 Reduction of functional cardiac Ca²⁺ ATPase protein content in test model.** Measurement of cardiac Ca²⁺ ATPase content in control and heterozygous animals (Mean ± SEM).
**Table 6 Breeding of tamoxifen-inducible cardiac Serca2 knockout mice** Breeding results for various combinations of Serca wt and flox alleles and the MCM (MerCreMer) transgene are are indicated. Both the Serca wt and flox alleles and the transgene segregate freely as indicated by similar distribution percentages.

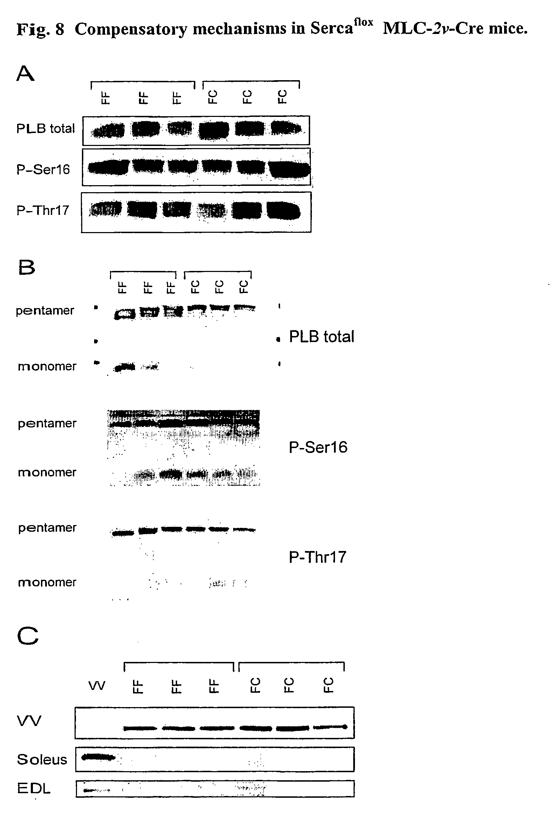

**Fig. 1** **Sequence alignment of mouse Serca 1, 2 and 3 protein.** Amino acid identity is designated by a dot. Gaps are designated by -. Amino acid substitutions in the Serca family proteins relative to mouse Serca1 protein are shown. Accession numbers for mouse Serca ATPases: mouse 1a, NP_031530; mouse 2a and 2b, NP_033852 and J131870; Serca3a, AAB04099; Serca3b, AAB04098; Serca3c, CAA75746.
**Fig. 2** **Sequence similarity of Serca2 proteins in mammalian species.** Amino acid identity is designated by a dot. Gaps are designated by -. Amino acid substitutions in the Serca2 proteins in other species relative to mouse Serca2 are shown. Accession numbers for Serca2 ATPases: mouse 2a and 2b, NP_033852 and J131870; rat 2a, P11507; rat 2b, NP_058986; human 2a, NP_001672; human 2b, NP_733765; human 2c, AAO47398; dog 2a, 046674; cat 2a, Q00779; pig 2a and 2b, P11607; rabbit 2a and 2b, P20647.

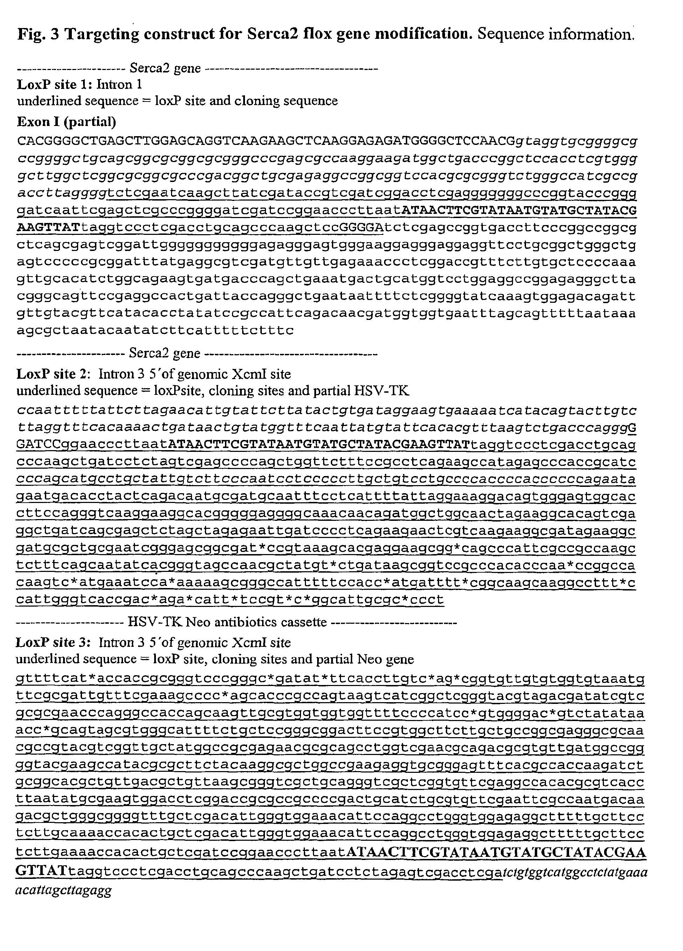

**Fig. 3** **Targeting construct for Serca2 flox gene modification.** Sequence information. Partial DNA sequence information of the targeting vector is given. Gaps in the sequence are given by lines and text of positioning in the *atp2a2* gene. Introns are in italics. Coding sequence is in capital letters. Extra inserted material (cloning sites, antibiotics cassettes and loxP sites) is underlined. LoxP sites are in bold. Elements are placed into atp2a2 gene intron 1, and intron 3 (2 positions). See figure 2 for schematic. The Serca2 gene sequence can be obtained from GenBank accession: NM_009722.

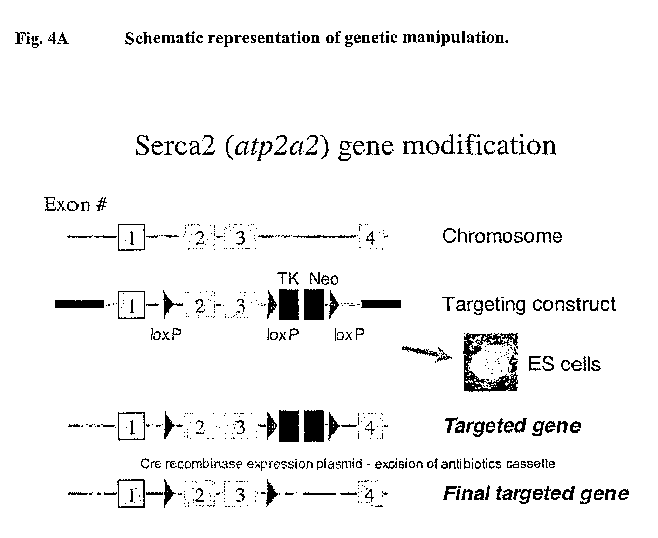

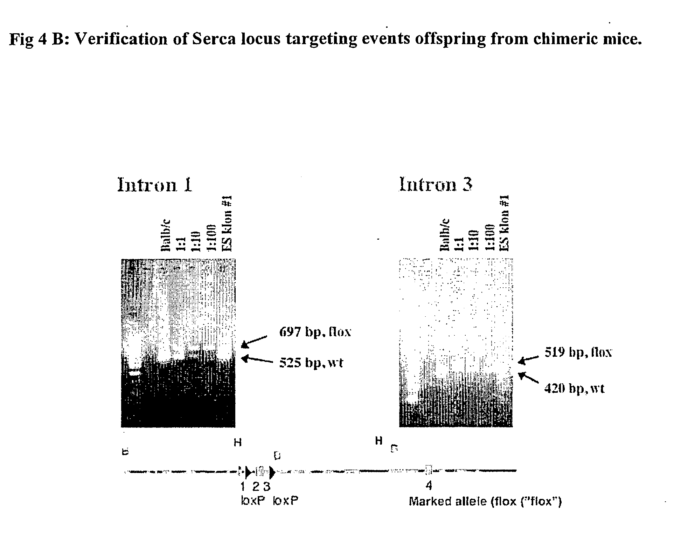

**Fig. 4****: A) Schematic representation of genetic manipulation.** A simple representation of the genetic elements introduced into the Serca2 gene introduced into the animal invention. B) Verification of Serca locus targeting events offspring from chimeric mice. Offspring from chimeric animals were genotyped by PCR. The intron 1 loxP site and intron 3 loxP sites were detected by primer pairs OL88/85 and OL86/87, respectively. DNA from Balb/C and from ES cell clone #1 were included as wild type and Serca2^{wt/flox} allele controls. Fractions indicate dilutions of tail DNA preparations

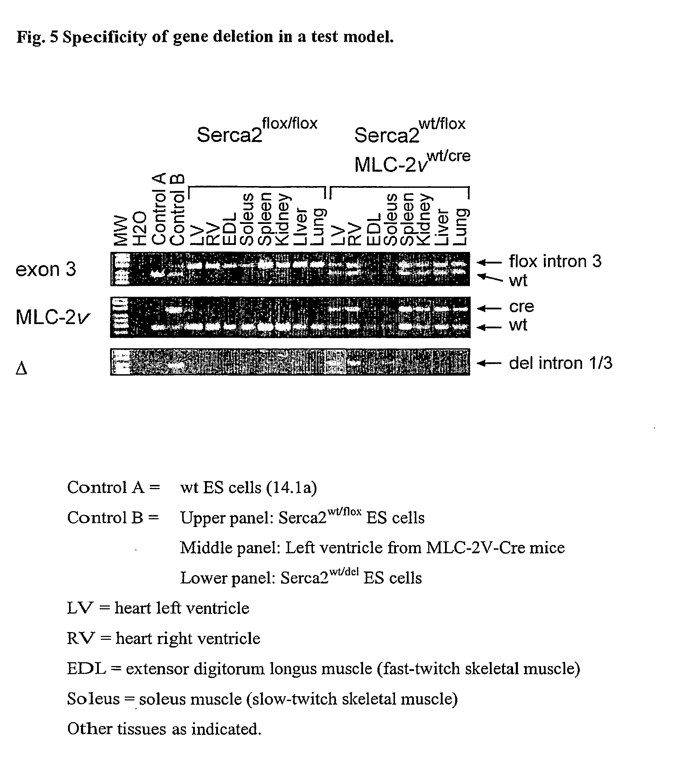

**Fig. 5** **Specificity of gene deletion in a test model.** Detection of Serca2 alleles in mouse tissues.

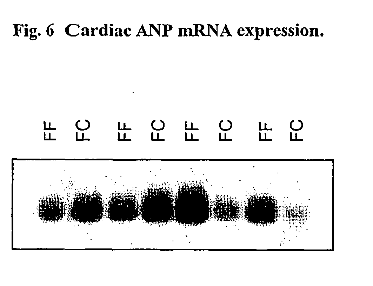

**Fig. 6** **Cardiac ANP mRNA expression.** The expression level of ANP was estimated by Northern analysis. Blot were sequentially probed with [32P]-dCTP-labelled probe for ANP. Genotypes: (Control) FF = Serca2^{flox/flox} (Cardiac heterozygous) FC = Serca2^{wt/flox} MLC-2v^{wt/cre}

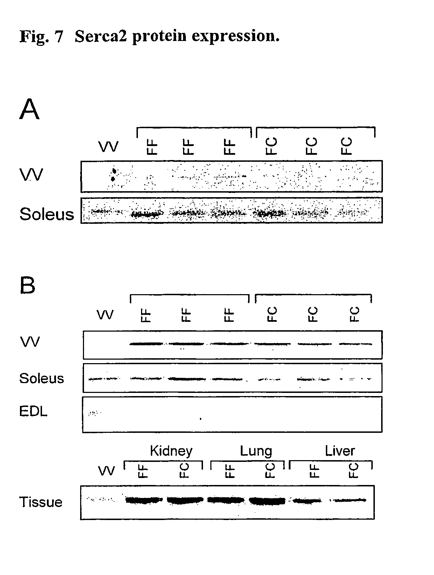

**Fig. 7** **Serca2 protein expression.** Total protein homogenates of the indicated tissues from control and cardiac heterozygous Serca2 mice were run on 8% SDS-PAGE gels and analyzed by Western blotting. Appropriate antibodies are given in the materials and methods section. Total lysate/lane: heart, 15µg, other tissues 40 µg. VV is heart lysate as positive control. Genotypes: (Control) FF = Serca2^{flox/flox} (Cardiac heterozygous) FC = Serca2^{wt/flox} MLC-2v^{wt/cre}
   A) Serca2a isoform in heart and soleus muscle
   B) Serca2b isoform in heart, soleus and EDL muscle
**Fig. 8** **Compensatory mechanisms in Serca2^{flox} MLC-2*v*-Cre** mice. Total protein homogenates from hearts from control and cardiac heterozygous Serca2 mice were run on SDS-PAGE gels (7% for NCX1, 15% for phospolamban) and analyzed by Western blotting. Gel loading was 10µg protein/sample except for detection of phosphoSer16 PLB (50µg/sample) and NCX1 in soleus + EDL (40 µg). Samples were denatured at 95 °C for 5-10 min (A, C) or at 37 °C for 15 min (B) to avoid complete disruption of PLB pentamers. Genotypes: (Control) FF = Serca2^{flox/flox} (Cardiac heterozygous) FC = Serca2^{wt/flox} MLC-2v^{wt/cre}
   Panel A) Total phospholamban, phospho-Ser16 PLB, and phospho-Ser16 PLB, monomeric
   Panel B) Total phospholamban, phospho-Ser16 PLB and phospho-Ser17 PLB, pentameric
   Panel C) NCX1. The band present in EDL muscle is not NCX1.

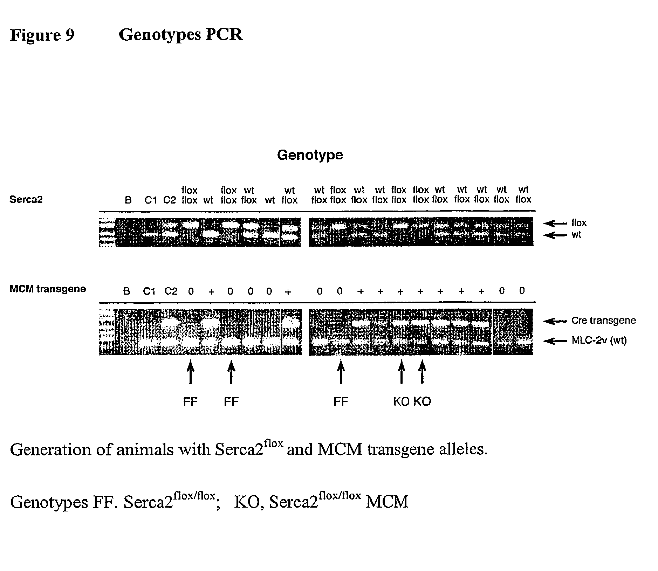

**Fig. 9** **Genotypes PCR.** Generation of animals with Serca2^{flox} and MCM transgene alleles. Genotypes FF. Serca2^{flox/flox}; KO, Serca2^{flox/flox} MCM

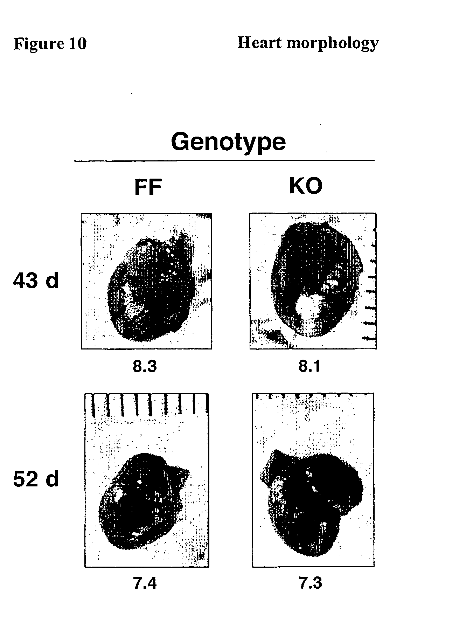

**Fig. 10** **Heart morphology.** Whole hearts from FF (ID 8.3 and 7.4) and KO (ID 8.1 and 7.3) animals were removed at day 43 or day 52 days after induction of gene excision with tamoxifen. Note the strongly enlarged left atrium in the KO animal (7.3) after 52 days.

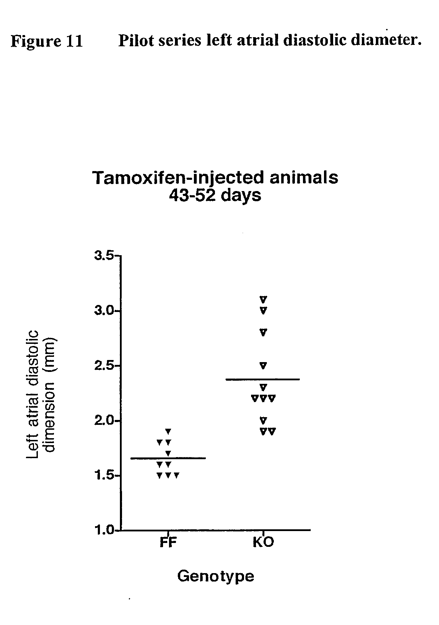

**Fig. 11** **Pilot series left atrial diastolic diameter.** Tamoxifen-injected mice were examined by echocardiography at the indicated time points. The left atrial diastolic dimension was the most sensitive parameter of heart dilatation. All KO animals had dilated left atria compared with FF controls

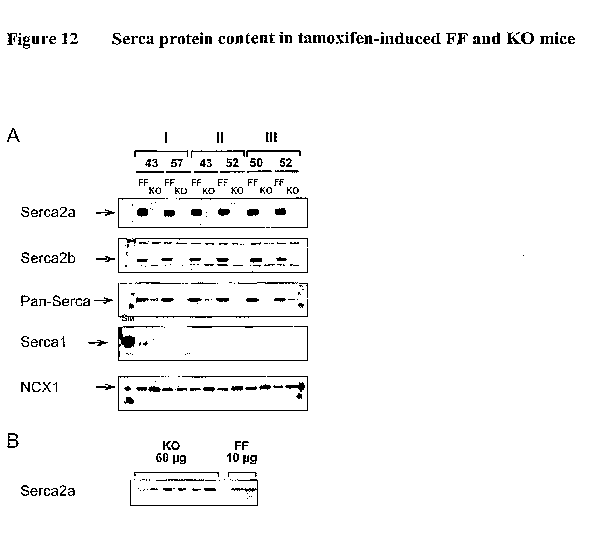

**Fig. 12** **Serca protein content in tamoxifen-induced FF and KO mice.** Roman numerals denote pilot experiments I, II and III. FF and KO denote genotypes Serca2^{flox/flox} and Serca2^{flox/flox} MCM respectively. Days after tamoxifen injection are noted at the top. A: Western blots with 20 µg total myocardial protein. Blots were probed with antibodies for Serca2a, Serca2b, pan-Serca (all Serca family members), Serca1 and NCX1 B: Western blot with 60 µg total protein for all 6 KO animals and 10 µg total protein for 2 FF control animals.

### DETAILED DESCRIPTION OF THE INVENTION

The present invention provides a genetically modified mouse having its genomic Serca ATPase gene modified by inserted recombination sites of heterogenous origin, said modification being homozygous. Said Serca ATPase gene modification may be present as one or several copies, with or without the co-presence of a wild-type Serca ATPase gene. Any Serca ATPase type or isoform can be employed in view of the high level of sequence similarity and conservation of function of the Serca family (See figure fig. 1), but preferably the ATPase gene is a Serca2 ATPase gene. Recombination sites can be inserted at any position adjacent or inside the Serca gene to cause complete or partial destruction of the gene when combined with a suitable recombinase. In general, any recombination sites suitable for eukaryotic cells can be used to carry out the present invention, like, for example, ftr or loxP recombination sites. Preferably, the recombination sites are of non-mammalian origin, and most preferably the recombination sites are loxP recombination sites. The recombination sites can be inserted in one or both alleles of the gene to cause a partial or complete destruction of Serca gene product activity. In a preferred embodiment of the present invention, the introduced loxP sites are placed such that both isoforms of the Serca2 genes (Serca2a and Serca2b) are disrupted in the tissue of interest when excision occurs. The shorter version Serca2a is expressed in the heart and in slow-twitch skeletal muscle, whereas the longer Serca2b protein is expressed in all tissues examined to date. Methods to introduce recombination sites into eukaryotic cells are known in the art.

The mouse of the current invention can further comprise a heterogenous recombinase, preferably of non-mammalian origin. This recombinase must be able to interact with the recombination sites of the modified Serca ATPase gene and effect inactivation of the gene. When terms like "destroyed", "disrupted", "destructed", "inactivated", or "excised" are used in relation to genes and the Cre-loxP system, or any similar system, it is herein meant to be construed as inactivation of a gene. In general, the recombinase can be of any type (e.g. FLP or Cre recombinase) provided it does not affect endogenous recombination sites, but preferably, the recombinase is a Cre recombinase.

The recombinase gene can be expressed in all cells of the vertebrate or only in certain tissues like, for example, tissue specific expression in skin, muscle, brain, kidney, or subgroups of these. In one specific embodiment of the present invention, a recombinase gene is expressed in the heart ventricle and in slow-twitch skeletal muscle. The recombinase gene can be exclusively, predominately, or modestly expressed in the tissue of interest compared to other tissues. In other words, the differential expression can vary greatly, but it is preferred that the recombinase is expressed exclusively or predominately in the tissue at stake. Furthermore, the recombinase gene can be expressed by induction, i.e. controlled by a regulatory nucleic acid sequence, preferably an inducible promoter or regulatory nucleic acid sequence that controls the expression of the gene, or extra fused gene material which controls the intracellular Cre recombinase location in the cell. An endogenous or exogenous inducing agent can effect the activation of this inducible promoter or regulatory sequence. Examples of the former are the tet system promoter, while examples of the latter are fusion of mutant estrogen receptor domain to the recombinase which confers tamoxifen inducibility. Methods to introduce a recombinase gene and its promoter or regulatory sequence into a eukaryotic cell are within the knowledge of a person skilled in the art.

Genes homologous or analogous to Serca ATPases exist in all vertebrates, and vertebrate model systems other than mice are consequently possible, e.g. fish, more particularly Zebra fish (*Branchydanio rerio*). Methods and procedures for handling of experimental animals, experimentally and ethically, are within the scope of the art.

Another aspect on the present invention is a eukaryotic cell having its genomic Serca ATPase gene modified by inserted recombination sites of heterogenous origin, said modification being homozygous, wherein said eukaryotic cell is not a human embryonic stem cell. The Serca ATPase gene, recombination sites, and regulatory nucleic acid sequences are as described *supra.* The cell is preferably of vertebrate or mammalian origin, more preferably of non-human mammalian origin, even more preferably of rodent origin, and most preferably of mouse origin. The cell may be of any particular type, except a human embryonic stem cell, for example, a cardiomyocyte, a keratinocyte, a neuron, an embryonic cell, etc. Most preferably, the cell is an embryonic cell, except a human embryonic stem cell. The cell may further comprise a non-native or heterogenous recombinase gene, more preferably a non-mammalian recombinase gene, such as a FLP or Cre recombinase gene. Preferably, the recombinase gene is a Cre recombinase gene. Methods for transfecting or transforming cells, cloning genes and genetically modifying genes are well known within the art.

Yet another aspect of the present invention is a gene encoding a Serca ATPase modified by inserted recombination sites, wherein said recombination sites are heterogenous to said gene.. The Serca ATPase gene, recombination sites, and regulatory nucleic acid sequences are as described *supra.* Suitable recombination sites can be inserted at any position adjacent or inside the Serca gene to cause complete or partial inactivation of the gene when present in a host cell. Any types of recombination sites can be used provided endogenous recombinases do not bind and effect recombination at the site when present in a host cell. Preferably, the recombination sites are of non-mammalian origin. According to a most preferred embodiment, the recombination sites are loxP recombination sites. The ATPase gene, which is to be modified, is preferably a Serca2 ATPase, and most preferably as listed in Genbank SEQ ID NM_009722. According to a most preferred embodiment, said Serca ATPase is modified as set forth in SEQ ID 1-3 or figure 3. Here, a modification of the flanks of the Serca2 gene, as employed in a most preferred embodiment, is depicted. The gene sequence of the Serca gene can be degenerated as a result of the redundancy of the genetic code. Also, codons can be changed with codons encoding similar amino acids. Methods necessary to enable the practice of the present invention are known within the art.

Another aspect of the present invention is a vector comprising the above Serca ATPase gene. The vector may be of any type appropriate for the task, for example, a shuttle vector, vectors for replication in eukaryotes or prokaryotes only, yeast vectors, etc. In a preferred embodiment the vector is based on pBluescript II KS.

Yet another aspect of the present invention is a method for screening a compound or a mixture of compounds for activity against defective Ca²⁺ handling, comprising the following steps:
- inducing expression of the recombinase, and with that inactivation of the Serca ATPase gene, in the mouse according to the present invention and
- administrating the compound or a mixture of compounds to said mouse before and/or after the induced inactivation of the Serca ATPase gene and
- detecting whether the induced defective Ca²⁺ handling is normalized by the administration of said compound or mixture of compounds.

The Serca ATPase gene and mouse are as described above and the Serca ATPase gene is inactivated by means of expressing a recombinase, also as described above. Parameters observed might, for example, be improvement in general condition, alleviation of symptoms, reduced lethality, and improvement of heart contractility.

The present invention is a valuable tool for studying (1) defective Ca²⁺ handling in a mouse and (2) heart failure in mouse. (1) The first method comprises the steps of inducing recombination and inactivation of a Serca ATPase gene in a mouse as described *supra.* This may be achieved by introducing a recombinase gene as described above into a genetically modified mouse having its genomic Serca ATPase gene modified by inserted recombination sites of heterogenous origin, said modification being homozygous; and further induce expression of the recombinase gene to effect Serca inactivation. Introduction of the gene can be done, for example, at the embryonic level by embryo fusion or by gene-therapeutic methods like exposing a live animal to a modified adenovirus carrying a suitable recombinase. In the latter case, the virus can then be targeted to specific tissues of the animal. Traditional mating is, however, a preferred method for producing animals whose cells, all or in selected tissues, comprise the above described modified Serca gene and an inducible recombinase with affinity for the recombination sites employed. The gene introduction step can of course be omitted in cases where suitable model animals already have been produced. The Serca ATPase gene is preferably of type 2 and the recombination sites can be inserted at any position adjacent or inside the Serca gene to cause complete or partial destruction of the gene in combination with a suitable recombinase. Preferably, the recombination sites are of non-mammalian origin. Most preferably, the recombination sites are loxP recombination sites. The recombination sites can be inserted in one or both alleles of the gene to bring about partial or complete destruction of Serca gene product activity. Methods to introduce recombination sites into eukaryotic cells are known in the art. The recombinase gene is preferably of non-mammalian origin, and most preferred a Cre recombinase. The recombinase is preferably controlled by an inducible promoter sequence or another inducible regulatory sequence. It is preferred that the recombination gene is only, or predominantly, expressed in specific tissues, as described above. This can be any tissue, but most preferably heart tissue. (2) The second method comprises the steps of inducing recombination and inactivation of a Serca ATPase gene in heart tissue. This may be achieved by mating a mouse with the above described modified Serca ATPase gene with a mouse partner, whose cells comprise a suitable recombinase gene expressed in heart tissue as described above; and further stimulate expression of the recombinase gene to induce Serca inactivation. However, any type of method can be used to produce animals with heart tissue comprising both a modified Serca gene and a nonnative or heterogenous recombinase. These methods include for example gene therapy, embryo fusion, etc. The step of mating or gene introduction can of course be omitted in cases where suitable model animals already have been produced. The recombinase gene is preferably controlled by an inducible promoter sequence or another inducible regulatory sequence, for example a tamoxifen-inducible promoter as described *supra.* Depending on the modification of the particular Serca ATPase; for example, whether the destruction is partial or complete; Ca²⁺ handling in heart tissue is partially or fully disturbed by the tissue specific induction of the recombinase. The reduced Ca²⁺ pumping ability, depending on the level of reduction, will lead to effects varying from decreased heart contractility to massive heart failure. Preferably, a genetically modified mouse having its genomic Serca ATPase gene modified by inserted loxP sites, said modification being homozygous, is mated with a mouse partner, all of whose cells comprise a Cre recombinase, to produce progeny with cells comprising both said Serca2 gene and Cre recombinase gene. Preferably, a mouse; all of whose cells comprise a Cre recombinase, but which is predominantly or only expressed in the heart ventricle and slow-twitch skeletal muscle; is mated with another mouse having its genomic Serca ATPase gene modified by inserted loxP sites, said modification being homozygous.

The expressions "introducing a gene into a vertebrate" or "introducing a gene into a vertebrate" are herein intended to mean any method for producing animals whose cells comprise both a recombinase and a modified Serca gene, both as described above. Examples of such methods are mating, gene therapy and embryo fusion.

Methods, practices and ethics related to gene therapy, mating and embryo fusion, as well as scientific experiments on non-human laboratory vertebrates or mammals are known by a person skilled in the art. Likewise, dosage and administration of a drug or chemical for induction of recombinase expression are known in the art.

### Examples of applications of Serca2^{flox} animals

### Serca dysfunction and heart failure

The non-human animal all of whose cells comprise a modified Serca ATPase gene can be bred with animal lines carrying the Cre recombinase expressed in the tissue of interest. As for the heart failure research, available animal Cre lines may direct Serca2 gene inactivation specifically in the heart in adult animals, by induction with tamoxifen (Sohal, Nghiem et al. 2001). This particular combination would provide a genetic means of inducing heart failure in a standardized experimental animal model. This model should be applicable for large-scale therapeutic drug screening and development. The major disadvantage of traditional animal models where heart failure is induced by operative procedures is the difficulty of standardizing operative procedures and their effects on the animals, and the difficulty of large-scale experiments required for therapeutic drug development.

The specificity of Serca2 gene inactivation in live mice is determined by the properties of the Cre recombinase-expressing mouse to which it is mated. For example, mice expressing the Cre recombinase specifically in the heart, brain, liver, lymphoid system or keratinocytes have been made, and can be bred to Serca2^{flox} mice to inactivate the Serca2 gene in those particular organs or tissues of interest.

The major application for Serca2^{flox} mice would be to generate mouse models for human diseases/disorders involving the Serca2 gene by breeding with appropriate recombinase-expressing mice. In the case where the mouse model reflects the phenotype of the human disorder, the mouse model should be a valuable test model for drug development. Inactivation of the Serca2 gene in the heart may induce heart failure, while inactivation of the Serca2 gene in keratinocytes may induce dermatological conditions. Furthermore it is expected that inactivation of the Serca2 gene in the brain may induce neurological dysfunction and inactivation of the Serca2 gene in the immune cells may induce immunological dysfunction.

### Skeletal muscle Serca dysfunction in heart failure

In congestive heart failure (CHF) patients, skeletal muscle fatigue is a prominent phenotype. Both fast-twitch and slow-twitch muscles seem to become slower, which may be linked to slower intracellular Ca²⁺ cycling (Lunde, Sjaastad et al. 2001; Lunde, Verburg et al. 2002). The neurohumoural stimuli for this change are unknown, but it has been reported that several cytokines are increased in these patients. Thus, the changes seen in skeletal muscles during CHF are in part secondary to inactivity, but may also be a consequence of altered Ca²⁺ metabolism, as seen in the myocardium. Our invention would provide a experimental model for this problem.

### Human dermatological disorders

In humans, mutations in the Serca2 gene have been linked with the skin disorders keratosis follicularis (Dariers's disease) and *acrokeratosis verruciformis* (Dhitavat, Macfarlane et al. 2003 & Hovnanian 2004). Darier's disease (DD) (keratosis follicularis) in humans is an autosomal dominant skin disorder characterized by loss of adhesion in epidermal cells and abnormal keratinization (Cooper and Burge 2003 & Hovnanian 2004). Current treatment is rudimentary. The Serca2 mutations found in Dariers'patients result in reduced Serca2 function, Ca²⁺ sensitivity or protein expression level in a heterologous expression assay in HEK-293 cells (Dode et al, 2003). However, there is no formal proof that the identified mutations result in loss of function and the observed phenotypes. A mouse model to test for the loss of Serca2 gene function in skin would therefore be necessary to prove the link between genotype and phenotype. Our invention can be bred such that the Serca2 gene is inactivated only in keratinocytes. Such an animal would be the only available animal model for these diseases and for drug development.

### Other areas

Ca²⁺ signaling is biologically very important in most tissues, particularly in the brain, immune cells and secretory cells. However, Serca2 function is poorly characterized in these organs and tissues. The Serca2^{flox} mouse could be used to generate mouse models to study Serca2-dependent Ca²⁺ handling functions in these cell types.

A null mutation in the Serca2 gene (*atp2a2*) in mice is embryonic lethal, and demonstrates the necessity of this protein (Periasamy, Reed et al. 1999). Mutations in the human *atp2a2* gene have been linked to dermatological disorders (Cooper and Burge 2003; Dhitavat, Macfarlane et al. 2003), and it is a matter of debate whether some of these mutations also are linked to neuropsychiatric disorders (Jacobsen, Lyons et al. 1999; Jacobsen, Franks et al. 2001; Wang, Yang et al. 2002).

It should also be mentioned that fish, particularly Zebra fish, is a promising and convenient model system to carry out the above experiments. Experimental results from such model systems can be directly applicable to higher vertebrates, like mammals, particularly human beings.

Having now fully described the present invention in some detail by way of illustration and example for purpose of clarity of understanding, it will be obvious to one of ordinary skill in the art that same can be performed by modifying or changing the invention with a wide and equivalent range of conditions, formulations and other parameters thereof, and that such modifications or changes are intended to be encompassed within the scope of the appended claims.

### MATERIALS AND METHODS

**Animal husbandry and breeding:** Animals were housed in accordance with the Guide for the Care and Use of Laboratory Animals (NIH Publication No. 85-23, revised1996). Mice were housed in M2 cages with Bee Kay bedding (Scanbur BK) in 55% humidity on a 12 h light/dark cycle. Food pellets (mouse + rat standard ex., Scanbur BR) and water was freely available.

Serca2wt/flox mice and MLC-2v-Cre knockin mice (Chen, Kubalak et al. 1998) were backcrossed onto B6/J for n=3 and n=1, respectively before interbreeding. All genotyping was performed on 5 mm tail clips (Torres and Kühn 1997) or 1 mm ear punches (Kappler & Marrack Research Laboratory, unpublished protocol) by PCR. Seventeen to 22 week animals of both sexes and genotypes specified in the text were used for basic functional characterization.

**Basic in vivo cardiac function:** Animals were prepared essencially as described (Woldbaek, Hoen et al. 2002). Briefly, animals were anaestized with i.v.tail injections of 0.2 mL propofol (10 mg/ml) and intubated with a 20-gauge i.v. cannula inserted into the trachea. The animals were ventilated closed chest in the supine position on a rodent ventilator (Model 874 092, B. Braun, Melsungen, Germany) with a mixture of 2% isofluran and 98% oxygen.

**Echocardiography and hemodynamic pressure measurements.** Measurements were performed as described (Finsen 2004). In short, mice were anaestetized, intubated and connected to a rodent ventilator using 2% isoflurane. Two-dimentional, M-mode and Doppler echocardiography was performed using a i13L 13MHz linear array transducer (GE) designed for the examination of small rodents. Data were analyzed with EchoPac PC software (GE). Hemodynamic measurements were performed sequencially with a 1.4 F Millar micro-tipped pressure transducer catheter (SPR-671, Millar Instruments, Houston, TX) advanced into the left ventricle. Data from 1-2 respiration cycles (10-15 consecutive beats) were used to calculate heart rate (HR), left ventricular systolic pressure (LVSP), left ventricular end-diastolic pressure (LVEDP), the maximal positive (LVd*P*/d*t*ₘₐₓ) and negative derivatives (LVd*P*/d*t*ₘᵢₙ) of the left ventricular pressure.

**Ca2+ ATPase vesicle activity:** Vesicular Ca2+ ATPase pumping in and out of SR vesicles was assessed by a fluorometric method.

**Ca²⁺ ATPase content:** Total Ca²⁺ ATPase in mouse heart left ventricles was measured by an ATP-binding assay in tissue homogenates as described elsewhere (Everts, Andersen et al. 1989; Holt and Christensen 1997).

**Cell lines:** The mouse embryonic stem cell line ES14.1a (stock at the KTTC, Karolinska Institute, Stockholm, Sweden) was used for genetic manipulations.

**Construction of Serca2^{flox} mice:** All genetic manipulations were performed using standard molecular biology techniques. The Serca2 *(atp2a2)* gene was cloned from a commercial genomic mouse lambda phage library **(129 SVJ from Stratagene).** A targeting vector, based on pBluescript II KS, containing 34 bp loxP sites (5'-ATA ACT TCG TAT AAT GTA TGC TAT ACG AAG TTA T-3') placed into appropriate positions in *atp2a2* gene fragments and antibiotics selection genes was assembled by standard cloning techniques. The targeting vector was named pSerca2T. All manipulations were confirmed by DNA sequencing (see figure 3). The targeting vector was introduced into mouse embryonic stem cells (ES cells) by standard techniques (electroporation). ES cells carrying homologous recombination events with correctly placed loxP sequences were enriched by antibiotics selection procedures, and identified by Southern hybridization techniques and PCR. The antibiotics selection genes were then excised by a second round of electroporation with a Cre recombinase-expressing plasmid. Correct clones were again identified by Southern blotting and PCR. The final result of the genetic manipulations was that two loxP sites and additional necessary cloning and restriction sites were introduced into 2 separate introns in the *atp2a2* gene. The modification is outlined in figure 4.

Correctly genetically modified ES cells carrying 2 loxP sites in one allele of the *atp2a2* gene were then used to generate genetically modified mice (Serca2^{flox} mice) carrying these loxP recombination sites. The loxP sites are placed such that the *atp2a2* gene is converted into a null allele when the Cre recombinase is appropriately expressed in the organ or tissue of interest. Subsequent production of all isoforms of Serca2 Ca²⁺ATPase protein is reduced or eliminated in the tissue of interest

**Mice:** The mouse line was backcrossed onto the B6/J background (N10), and bred to homozygosity.

**Northern blotting:** Gene expression in heart left ventricles was analyzed basically as described (Semb, Lunde et al. 1998). Poly A⁺ RNA was captured onto Dynabeads oligo(dT)₂₅ according to the manufacturers protocol (Dynal AS, Norway). Samples (10 µg mRNA) were size-fractionated on 1% agarose gels and. Filters were probed with cDNA framents for for Serca2 (pRH39, EcoRI 2.2 kb insert)(Lompre, de la Bastie et al. 1989), ANP (pGANP-1, PstI 650 bp insert) (Glembotski, Oronzi et al. 1987) and GAPDH (1.3 kb insert) (a kind gift from Hans Prydz).

**PCR detection:** Different alleles of the Serca2 gene (Serca2 wt, flox or deletion) and the MLC-2v gene (wt and cre knock-in) were detected by standard PCR reactions with annealing temperature of 55 °C for 25-30 cycles and Amplitaq Gold on an ABI 9600 thermocycler (Applied Biosystems).

Primers were as follows:
OL84 5'- cca agg aag atg gct gac c - 3'
OL85 5'- cat cga cgc ctc ata aat cc - 3'
OL86 5'- tct tca taa cac acg cca att t - 3'
OL87 5'- ccc ttt gct gcc aat taa cta tt - 3'
OL88 5'- acc tct agg ggt ctc gaa tca - 3'
OL102 5'- aag ttg aat aac cgg aaa tgg ttt - 3'
OL103 5'- tgt tat aag caa tcc cca gaa atg - 3'
OL104 5'- agg ctc ctc gaa ctc tcc ag - 3'
OL105 5'- gta aga gag ctt ccc tcc tcc tt - 3'

Primer combinations and PCR amplification product sizes are given below:

| **Position** | **Primer pair** | **PCR product** |
|---|---|---|
| Serca2 intron 1 | OL84 / OL85 | wt: 247 bp |
| | | flox: 419 bp |
| Serca2 intron 1 | OL88 / OL85 | flox: 318 bp |
| Serca2 intron 3 | OL86 / OL87 | wt: 282 bp |
| | | flox: 381 bp |
| Serca2 deletion | OL88 / OL87 | 324 bp |
| Cre (knockin) | OL102 / OL103 | 340 bp |
| MLC-2v wt | OL104 / OL105 | 183 bp |

**Statistical analysis:** The data are expressed as mean values +/-S.E.M. Statistical significance was calculated using Student's unpaired t-test (Graphpad Prism, 4.01). P values <0.05 were considered significant.

**Tissue material:** Immediately after conclusion of the *in vivo* measurements, the mice were sacrificed with excision of heart, lung, liver, kidney, spleen, soleus and extensor digitorum longus (EDL) muscles. Hearts and lungs were blotted dry and immediately weighed. All organs were flash frozen in liquid nitrogen.

**Western blotting:** Mouse tissues were homogenized in ice-cold buffer (210 mM sucrose, 2mM EGTA, 40mM NaCl, 30mM Hepes, 5mM EDTA, pH 7.4) supplemented with a protease inhibitor cocktail (Complete EDTA-free, Roche Diagnostics, Mannheim, Germany) essencially as described (Ploug, Wojtaszewski et al. 1993). Homogenization buffer was also supplemented with phosphatase inhibitors (phosphatase inhibitor cocktail 1, Sigma) where needed. SDS was added to 1% final concentration, and the lysate was incubated for 15 minutes at room temperature before aliquoting and freezing at -80 °C. Protein content was measured by a bicinchoninic acid assay (Pierce 23235) using BSA as standard protein. Samples were electrophoresed in SDS-PAGE gels (8% or 15%) and transferred to 0.45µm PVDF membranes by standard procedures. Membranes were blocked in 5% skim milk in TBS-T (Amersham Life Sciences) or in an alternative BPPT blocking solution (5% BSA, 1% polyvinylpyrrolidone-10, 1% polyethyleneglycol, MW 3500, 0.2% Tween 20 in 2xTBS) (NanoTools Antikörpertechnik, info@nanotools.de) where indicated. The following antibodies were used for protein detection: monoclonal antibodies against Serca2a (MA3-919), phospholamban PLB (MA3-922) (all Affinity Bioreagents), polyclonal anti-Serca2b antiserum(Campbell, Wuytack et al. 1993) (kind gift from Frank Wuytack), anti-NCX1 (Thomas, Sjaastad et al. 2003), anti-phospho-Ser16-PLB and anti-phospho-Thr17-PLB (both Badrilla). Blots were incubated with appropriate HRP-conjugated sheep anti-mouse IgG or donkey anti-rabbit IgG secondary antibodies (Amersham Biosciences) and developed with ECL or ECLplus reagents (Amersham Biosciences). Images were acquired in a LAS-1000 CCD detection system (Fuji film). All membranes were post-immunoblotting stained with Coomassie Brilliant Blue R250, and regions in each lane were used for normalization of protein loading on the membrane.

### EXAMPLES

### Example 1: Generation of cardiac-specific Serca2^{wt/flox} heterozygous mice

Serca2^{flox} mice were intercrossed with MLC-2v Cre knock-in mice (Chen, Kubalak et al. 1998; Minamisawa, Gu et al. 1999) in which the Cre recombinase is expressed in the heart ventricle and in slow-twitch skeletal muscle with no detectable basal expression in other tissues. The Serca2^{flox} and MLC-2v Cre knock-in mice were at generation N3 and N3 onto the B6/J background before interbreeding. In the final breeding between Serca2^{flox/flox} and Serca2^{wt/flox} MLC2v^{wt/cre} animals, we were unable to obtain offspring with the expected homozygous "cardiac knockout" genotype (Serca2^{flox/flox} MLC2v^{wt/cre}) (table 1). More than 100 offspring were genotyped. In the six mating pairs (with alternation of parental genotypes), we could detect offspring with only 2 of the 4 expected genotypes (Serca2^{flox/flox} MLC2v^{wt/wt} and Serca2^{wt/flox} MLC2v^{wt/cre}). Since litter sizes were normal and the wild type genotype also was missing, the genotype distribution indicated that the Serca2 and MLC2-v gene loci did not segregate freely. The breeding result could be explained by a lack of chromosomal crossover between the Serca2 (*atp2a2*) and MLC-2v (*myl2*) loci. During this breeding period, the MLC-2v gene was assigned a chromosomal position in close proximity to Serca2 on mouse chromosome 5, cytoband F (approx. distance 630 000 nt). In conclusion, the linkage between the Serca2 (atp2a2) and MLC-2v (myl2) genes on chromosome 5 is for practical purposes 100%.

### Example 2: Characterization of cardiac-specific Serca2^{wt/flox} heterozygous mice

### Specific gene inactivation

The tissue specificity of the Serca2 gene inactivation was confirmed by PCR analysis of DNA from mouse tissues (Fig. 5). In Serca2^{wt/flox} MLC-2v^{wt/cre} double heterozygous mice, deletion of one copy of the Serca2 gene was detected only in the heart and in slow-twitch skeletal muscle (soleus). No Serca2 deletion PCR product was detected in EDL (fast-twitch) muscle, liver, lung, kidney or spleen. No excision was detected in control littermates (Serca2^{wt/flox} MLC-2v^{wt/wt}). This result was expected from the expression of Cre recombinase from the MLC-2v gene locus in this particular cross.

### Animal physiology

The animal age, heart, lung and body weights were measured in Serca2^{wt/flox} MLC-2v^{wt/cre} double heterozygous mice and in control littermates (Serca2^{wt/flox} MLC-2v^{wt/wt}) (Table 2). In total, hearts and organs were examined from 71 animals. There were no gross anatomical differences. Overall, there was no significant difference between the two genotypes in animal size, heart and lung weight, or in the ratios of heart weight/body weight or lung weight/body, indicative of no overt disease in the double heterozygous animals. As expected, females had significantly lower body weight, heart weight and lung weight than males. However, no significant differences were detected between the genotypes within each sex.

### Serca2 expression in cardiac heterozygous Serca2 animals

The amount of functional Ca²⁺ATPase enzyme in hearts from both genotypes was measured by an ATP-binding assay (table 5). In Serca2^{wr/flox} MLC-2v^{wt/cre} double heterozygous mice, the content of ATPase in the heart was reduced by 32% in 11 week old mice (n=2 for each group), and by 28% in 19-week old mice compared with control Serca2^{flox/flox} MLC-2v^{wt/wt} littermates .

The protein content of both Serca2a and Serca2b isoforms were examined in both left ventricle and in slow-twitch soleus muscle by Western blotting with isoform-specific antibodies. In the left ventricle, Serca2a was reduced by 28% and 26% in double heterozygous Serca2^{wt/flox} MLC-2v^{wt/cre} mice (figure 7, panel A) compared with control Serca2^{flox/flox} MLC-2v^{wt/wt} littermates. A parallel decrease in cardiac Serca2b protein was also detected, 49% in double heterozygotes compared to control animals (panel b). In the soleus muscle, Serca2a and Serca2b protein was reduced by 25% and 35% in the double heterozygotes compared to control animals (figure 7, panel B). In other tissues such as lung, spleen, liver and kidney, there was no decrease in Serca2b expression in the double heterozygous animals relative to controls (figure 7, panel B, lower part). Taken together, the amount Serca2a and Serca2b proteins were reduced specifically in the "cardiac Serca2 heterozygous" animals in the heart and in slow-twitch soleus muscle, but not in other tissues.

### In vivo heart function

Basal *in vivo* heart function was first assessed by echocardiography (table 3). Overall heart dimensions, aorta dimensions, flow rates and cardiac output was not significantly different between the cardiac heterozygous Serca2 (Serca2^{wt/flox} MLC-2v^{wt/cre}) and control animals (Serca2^{wt/flox} MLC-2v^{wt/wt}). There were no indications of cardiac dysfunction in either genotype.

Haemodynamic pressure measurements were then sequentially performed on each animal with a pressure transducer inserted into the left ventricle. Again, no differences were observed between the two genotypes, nor any indications of cardiac dysfunction (table 4).

Atrial natriuretic factor is readily induced in stressed, hypertrophic or dysfunctional hearts. No differences in ANP expression between the two genotypes was detected, arguing against the hearts being in a stressed state at a subclinical level (figure 6).

### Compensatory mechanisms in Serca2 expression in cardiac heterozygous animals

The total level of phospholamban was unchanged, as evaluated by PLB monomers under reducing conditions (figure 8). Likewise, there was no shift in Ser-16 or Thr-17 phosphorylated monomers in double heterozygous animals relative to controls. However, there seems to be shift in the content of pentameric PLB protein in the heterozygotes compared with flox controls.

In the heart, NCX1 RNA expression was increased by 40% (figure 8, panel C). In EDL and soleus muscle, there was no detectable NCX1 immunoreactivity. This is in keeping with data from the Serca2 heterozygous mouse (Periasamy, Reed et al. 1999), and provides an explanation for compensatory pathway for removing Ca²⁺ from the cytosol out of the cell via NCX1, since the expression level of Serca2, and removal of Ca²⁺ into the SR, is reduced

### Summary

In our model, the gene inactivation occurred only in the tissue of interest, as demonstrated in this particular test breeding of Serca2^{flox} mice of the invention with MLC-2v Cre mice. Serca2a was partially inactivated in the heart and in slow skeletal muscle of Serca2^{wt/flox} MLC-2v^{wt/cre} double heterozygous mice as shown by PCR, ATPase and protein measurements.

In conclusion, the invention can be used to generate standardized animal models with defective Ca²⁺ handling in the tissue of organ of interest, and thus be useful in biomedical research and therapeutic drug development.

### Example 3: Generation of cardiac tamoxifen-inducible Serca2 knockout mice

We were, as described in Example 1, unable to obtain cardiac Serca2 knockout mice by breeding Serca2^{flox} mice with MLC-2v-Cre mice due to locus linkage. Serca2^{flox} mice were the bred to transgenic MerCreMer mice, which express the Cre recombinase in the heart under control of the alpha-myosin heavy chain promoter (Sohal, Nghiem et al. 2001). In addition, the presence of Cre recombinase in the nucleus, and thereby activity, is controlled by tamoxifen.

### Example 4: Induction of Serca2 inactivation

Cardiac Serca2 knockout mice will be produced by injection of Serca2^{flox/flox} MerCreMer with tamoxifen as described (Sohal, Nghiem et al. 2001). The animals will be characterized as the heterozygous animals of Examples 1 to 2. In addition, the forward and reverse mode functions of Serca2 ATPase in cardiac SR vesicles will be measured.

One predicted phenotype of this cardiac restricted Serca2 knockout mouse can be full compensation because other proteins are able to sufficiently handle the Ca²⁺ movements in the cardiomyocyte (e.g. NCX1). The other extreme phenotype may be sudden death due to a collapse in the electrochemical balance in the cardiomyocytes (arrhythmic phenotype). In this respect, the animals are evaluated by ECG to detect arrhythmic activity. We anticipate that there will be a time window in which Serca2 protein progressively is reduced. The heart will first have sufficient time to develop compensatory mechanisms, but then progress into a failing state as the Serca2 function is severely reduced. The heart may display a hypertrophic phenotype with activation of cytokine systems and other signal transduction pathways. This model would then mimic the compensated and failing stages of heart failure in humans. Given the latter scenario, the endpoint will be death. This is a "hard" endpoint ideal for the invention's proposed use as a tool for developing improved drugs and therapies against heart failure. Also, another feasible feature will be the extended time window which will permit time for conducting the experiment as well as monitoring.

### Example 5: Generation and characterization of cardiac tamoxifen-inducible Serca2 knockout mice

Serca2^{flox} mice were the bred to transgenic MerCreMer (MCM) mice, which express the Cre recombinase in the heart under control of the α-myosin heavy chain promoter (Sohal, Nghiem et al. 2001). This particular version of Cre recombinase is fused to two copies of a mutant estrogen receptor ligand binding domain which bind the estrogen antagonists tamoxifen and 4-OH tamoxifen. The expressed transgene is localized in the cell cytoplasm in cardiac myocytes. Upon binding to tamoxifen, the transgene enters the nucleus and permit excision of the Serca2^{flox} allele. Thus, excision of the Serca2^{flox} allele is controlled both by tissue specificy and the presence of tamoxifen.

### Pre knock-out physiology

All breeding combinations of Serca2^{flox} and transgenic MCM mice generated the expected genotypes at the expected frequencies (table 6 and figure 9). Serca2^{flox/flox} MCM mice breed normally, and show no overt signs of cardiac dysfunction or other abnormal physiology.

### Example 6: Induction of Serca2 inactivation

### a. Serca2 gene inactivation

Tamoxifen (1 mg/100µl in peanut oil or sunflower seed oil) was administered i.p. to each animal at dosages of 1 mg/day for 5 days or for 2x5 days (weekdays).

### b. Morbidity and mortality

In the first pilot series, 4 mice, two of each genotype: Serca2^{flox/flox} (denoted FF) and Serca2^{flox/flox} MCM (denoted KO) received tamoxifen i.p. at 1 mg/day for 5 days. After 43 days one FF and one KO animal were examined and no overt signs of heart failure were seen. Hearts were removed for biochemical analysis. After 57 days the remaining KO animal died of severe heart failure with a severely enlarged left atrium, enlarged right ventricle, increased lung weight, fluid in the thorax and abdomen, whereas the FF mouse was completely normal. In the second pilot series using 4 animals, two of each genotype as above received tamoxifen i.p. at 1 mg/day for 2x5 days. At 43 days, one FF and one KO animal were screened by echocardiography as described below. The KO animal displayed a slight dilatation of the left atrium, but was otherwise normal, whereas the FF animal was completely normal. At 52 days, the remaining KO animal again developed severe heart failure as described above, whereas the FF animal was normal with no signs of heart failure (figure 10).

In a third trial series, 4 FF and 4 KO of various age (12-17 week weeks) received tamoxifen i.p. at 1 mg/day for 5 days. Animals were followed by non-invasive echocardiography at day 45, 48, 50 and 52 days (see below) until severe heart failure and termination of the animal. All KO animals developed severe cardiac failure within day 52 with dilation of the left atrium as noted above, increased lung weight, fluid in the thorax and abdomen, whereas the FF controls were completely normal.

### c. In vivo heart function

### Non-invasive echocardiography pilot series.

Animals at various ages were injected with tamoxifen as described above. They were analyzed by echocardiography at 45, 48, 50 and 52 days after tamoxifen injection. Data were analyzed with EchoPac PC software (GE). Because of variable anesthesia depth due to the mask, heart rate-dependent parameters were variable. The most consistent parameter was the left atrial diastolic dimension. KO animals developed enlarged left atria at 45 days post-injection which progressively worsened, whereas FF animals remained normal (Figure 11).

### d. Serca2 expression in cardiac Serca2 knockout animals

Serca2 content in the hearts from FF and KO animals from the first three pilot experiments (see above) were analyzed by Western blotting (Figure 12).

Blots were probed with isoform-specific antibodies to Serca2a and Serca2b, Serca1, pan-Serca and NCX1. In all 6 KO animals, Serca2a protein was almost undetectable, whereas Serca2 was readily available in all 6 FF animals, regardless of time point. The almost undetectable Serca2a level in KO animals was confirmed by Serca2b and pan-Serca antibodies. This extremely low Serca2 protein band may represent Serca2 expressed in other cells in the myocardium such as fibroblasts, in which gene excision should not occur. There was no compensatory induction of Serca1 (fast-twitch muscle ATPase) in the KO myocardium. All KO animals had increased NCX1 protein. Interestingly, both KO mice at 43 days post-injection had no prominent symptoms of heart failure even though Serca2 protein was almost undetectable in the myocardium of these animals. This observation strongly suggests that unknown mechanisms must compensate for the loss of Serca in the large movement of Ca²⁺ in cardiomyocytes during the beat-to-beat contraction cycle.

### e. Summary features of model

The mice displayed clinical signs of heart failure such as sluggishness, fluid in the thorax and abdomen at the most advanced stage, tachypnoe and poor general condition. The animals displayed dilated left atrium and right ventricle. At the late endpoint (48-52 days), left ventricular function was also compromised. There were no indications of hypertrophy in the myocardium.

The only other consistent observation in tamoxifen-injected males was swelling in the scrotum regardless of genotype. This observation is consistent with tamoxifen has effects in male mice, but is related to the genetic background (Nakai et al 1999) rather than the manipulation of Serca2 gene expression in the myocardium.

### f. Other comments and conclusion

This model should be suitable as a general heart failure model without the interference of hypertrophic processes. The animals also may be useful as a model for right-sided cardiac dysfunction.

### REFERENCES

Antoons, G., M. Ver Heyen, et al. (2003). "Ca2+ uptake by the sarcoplasmic reticulum in ventricular myocytes of the SERCA2b/b mouse is impaired at higher Ca2+ loads only." Circ Res 92(8): 881-7.
Arredouani, A., Y. Guiot, et al. (2002). "SERCA3 ablation does not impair insulin secretion but suggests distinct roles of different sarcoendoplasmic reticulum Ca(2+) pumps for Ca(2+) homeostasis in pancreatic beta-cells." Diabetes 51(11): 3245-53.
Campbell, A. M., F. Wuytack, et al. (1993). "Differential distribution of the alternative forms of the sarcoplasmic/endoplasmic reticulum Ca(2+)-ATPase, SERCA2b and SERCA2a, in the avian brain." Brain Res 605(1): 67-76.
Chen, J., S. W. Kubalak, et al. (1998). "Selective requirement of myosin light chain 2v in embryonic heart function." J Biol Chem 273(2): 1252-6.
Cooper, S. M. and S. M. Burge (2003). "Darier's Disease: Epidemiology, Pathophysiology, and Management." Am J Clin Dermatol 4(2): 97-105.
Dhitavat, J., S. Macfarlane, et al. (2003). "Acrokeratosis verruciformis of Hopf is caused by mutation in ATP2A2: evidence that it is allelic to Darier's disease." J Invest Dermatol 120(2): 229-32.
Dode L, Andersen JP, Leslie N, Dhitavat J, Vilsen B, and Hovnanian A. (2003). Dissection of the functional differences between sarco(endo)plasmic reticulum Ca2+-ATPase (SERCA) 1 and 2 isoforms and characterization of Darier disease (SERCA2) mutants by steady-state and transient kinetic analyses. J Biol Chem 278: 47877-47889.
Everts, M. E., J. P. Andersen, et al. (1989). "Quantitative determination of Ca2+-dependent Mg2+-ATPase from sarcoplasmic reticulum in muscle biopsies." Biochem J 260(2): 443-8.
Finsen AV, Christensen G, and Sjaastad I. (2004).Echocardiographic parameters discriminating myocardial infarction with pulmonary congestion from myocardial infarction without congestion in the mouse. J Appl Physiol.
Frank, K. F., B. Bolck, et al. (2003). "Sarcoplasmic reticulum Ca2+-ATPase modulates cardiac contraction and relaxation." Cardiovasc Res 57(1): 20-7.
Gelebart, P., V. Martin, et al. (2003). "Identification of a new SERCA2 splice variant regulated during monocytic differentiation." Biochem Biophys Res Commun 303(2): 676-84.
Glembotski, C. C., M. E. Oronzi, et al. (1987). "The characterization of atrial natriuretic peptide (ANP) expression by primary cultures of atrial myocytes using an ANP-specific monoclonal antibody and an ANP messenger ribonucleic acid probe." Endocrinology 121(3): 843-52.
Hasenfuss, G. and B. Pieske (2002). "Calcium cycling in congestive heart failure." J Mol Cell Cardiol 34(8): 951-69.
Holt, E. and G. Christensen (1997). "Transient Ca2+ overload alters Ca2+ handling in rat cardiomyocytes: effects on shortening and relaxation." Am J Physiol 273(2 Pt 2): H573-82.
Holt, E., T. Tønnessen, et al. (1998). "Mechanisms of cardiomyocyte dysfunction in heart failure following myocardial infarction in rats." J Mol Cell Cardiol 30(8): 1581-93.
Hovnanian A. (2004). Darier's disease: from dyskeratosis to endoplasmic reticulum calcium ATPase deficiency. Biochem Biophys Res Commun 322: 1237-1244, 2004.
Huke, S., L. H. Liu, et al. (2003). "Altered force-frequency response in non-failing hearts with decreased SERCA pump-level." Cardiovasc Res 59(3): 668-77.
Jacobsen, N. J., E. K. Franks, et al. (2001). "Exclusion of the Darier's disease gene, ATP2A2, as a common susceptibility gene for bipolar disorder." Mol Psychiatry 6(1): 92-7.
Jacobsen, N. J., I. Lyons, et al. (1999). "ATP2A2 mutations in Darier's disease and their relationship to neuropsychiatric phenotypes." Hum Mol Genet 8(9): 1631-6.
Ji, Y., M. J. Lalli, et al. (2000). "Disruption of a single copy of the SERCA2 gene results in altered Ca2+ homeostasis and cardiomyocyte function." J Biol Chem 275(48): 38073-80.
Ji, Y., E. Loukianov, et al. (1999). "Analysis of sarcoplasmic reticulum Ca2+ transport and Ca2+ ATPase enzymatic properties using mouse cardiac tissue homogenates." Anal Biochem 269(2): 236-44.
Lompre, A. M., D. de 1a Bastie, et al. (1989). "Characterization and expression of the rat heart sarcoplasmic reticulum Ca2+-ATPase mRNA." FEBS Lett 249(1): 35-41.
Loukianov, E., Y. Ji, et al. (1998). "Sarco(endo)plasmic reticulum Ca2+ ATPase isoforms and their role in muscle physiology and pathology." Ann N Y Acad Sci 853: 251-9.
Lunde, P. K., I. Sjaastad, et al. (2001). "Skeletal muscle disorders in heart failure." Acta Physiol Scand 171(3): 277-94.
Lunde, P. K., E. Verburg, et al. (2002). "Contractile properties of in situ perfused skeletal muscles from rats with congestive heart failure." J Physiol 540(Pt 2): 571-80.
Minamisawa, S., Y. Gu, et al. (1999). "A post-transcriptional compensatory pathway in heterozygous ventricular myosin light chain 2-deficient mice results in lack of gene dosage effect during normal cardiac growth or hypertrophy." J Biol Chem 274(15): 10066-70.
Nakai M, Uchida K, Teuscher C (1999). "The development of male reproductive organ abnormalities after neonatal exposure to tamoxifen is genetically determined". J Androl.;20:626-34
Nakayama, H., K. Otsu, et al. (2003). "Cardiac-specific overexpression of a high Ca2+ affinity mutant of SERCA2a attenuates in vivo pressure overload cardiac hypertrophy." Faseb J 17(1): 61-3.
Pan, Y., E. Zvaritch, et al. (2003). "Targeted disruption of the ATP2A1 gene encoding the sarco(endo)plasmic reticulum Ca2+ ATPase isoform 1 (SERCA1) impairs diaphragm function and is lethal in neonatal mice." J Biol Chem 278(15): 13367-75.
Periasamy, M. and S. Huke (2001). "SERCA pump level is a critical determinant of Ca(2+)homeostasis and cardiac contractility." J Mol Cell Cardiol 33(6): 1053-63.
Periasamy, M., T. D. Reed, et al. (1999). "Impaired cardiac performance in heterozygous mice with a null mutation in the sarco(endo)plasmic reticulum Ca2+-ATPase isoform 2 (SERCA2) gene." J Biol Chem 274(4): 2556-62.
Ploug, T., J. Wojtaszewski, et al. (1993). "Glucose transport and transporters in muscle giant vesicles: differential effects of insulin and contractions." Am J Physiol 264(2 Pt 1): E270-8.
Sande, J. B., I. Sjaastad, et al. (2002). "Reduced level of serine(16) phosphorylated phospholamban in the failing rat myocardium: a major contributor to reduced SERCA2 activity." Cardiovasc Res 53(2): 382-91.
Semb, S. O., P. K. Lunde, et al. (1998). "Reduced myocardial Na+, K(+)-pump capacity in congestive heart failure following myocardial infarction in rats." J Mol Cell Cardiol 30(7): 1311-28.
Shull, G. E., G. Okunade, et al. (2003). "Physiological functions of plasma membrane and intracellular Ca2+ pumps revealed by analysis of null mutants." Ann N Y Acad Sci 986: 453-60.
Sjaastad, I., O. M. Sejersted, et al. (2000). "Echocardiographic criteria for detection of postinfarction congestive heart failure in rats." J Appl Physiol 89(4): 1445-54.
Sohal, D. S., M. Nghiem, et al. (2001). "Temporally regulated and tissue-specific gene manipulations in the adult and embryonic heart using a tamoxifen-inducible Cre protein." Circ Res 89(1): 20-5.
Thomas, M. J., I. Sjaastad, et al. (2003). "Localization and function of the Na(+)/Ca(2+)-exchanger in normal and detubulated rat cardiomyocytes." J Mol Cell Cardiol 35(11): 1325-37.
Torres, R. M. and R. Keuhn (1997). Laboratory protocols for conditional gene targeting. Oxford ; New York, Oxford University Press.
Ver Heyen, M., S. Heymans, et al. (2001). "Replacement of the muscle-specific sarcoplasmic reticulum Ca(2+)-ATPase isoform SERCA2a by the nonmuscle SERCA2b homologue causes mild concentric hypertrophy and impairs contraction-relaxation of the heart." Circ Res 89(9): 838-46.
Wang, S. L., S. F. Yang, et al. (2002). "Darier's disease associated with bipolar affective disorder: a case report." Kaohsiung J Med Sci 18(12): 622-6.
Wuytack, F., L. Raeymaekers, et al. (2002). "Molecular physiology of the SERCA and SPCA pumps." Cell Calcium 32(5-6): 279-305.
Woldbaek, P. R., I. B. Hoen, et al. (2002). "Gene expression of colony-stimulating factors and stem cell factor after myocardial infarction in the mouse." Acta Physiol Scand 175(3): 173-81.
Woldbaek, P. R., T. Tonnessen, et al. (2003). "Increased cardiac IL-18 mRNA, pro-IL-18 and plasma IL-18 after myocardial infarction in the mouse; a potential role in cardiac dysfunction." Cardiovasc Res 59(1): 122-31

### Tables:

**Table 1**

| **Genotype distribution of breeding results for Serca2^{flox} and MLC-2v-Cre knock-in mice.** | | | | | |
|---|---|---|---|---|---|
| **Cross: Parental genotypes** Serca2^{wt/flox} MLC-2v^{wt/cre} X Serca2^{flox/flox} MLC-2v^{wt/wt} | | | | | |
| | | | | **Expected %** | **Expected %** |
| **Offspring** | **Genotype** | n=101 | % | (free segregation) | (no crossover) |
| Serca2 Flox heterozygous control | Serca2^{wt/flox} MLC-2v^{wt/wt} | 0 | 0 | 25 | 0 |
| Serca2 Flox homozygous control | Serca2^{flox/flox} MLC-2v^{wt/wt} | 44 | 44 | 25 | 50 |
| Cardiac Serca2 heterozygous | Serca2^{wt/flox} MLC-2v^{wt/cre} | 56 | 55 | 25 | 50 |
| Cardiac Serca2 knockout | Serca2^{fiox/flox} MLC-2v^{wt/cre} | 0 | 0 | 25 | 0 |

**Table 2**

| **Body and organ weights for Serca2 cardiac heterozygous and control mice.** Age, body, heart and lung weights of control and heterozygous animals (Mean ± SEM). | | | | |
|---|---|---|---|---|
| **By genotype** | **FF** | **n** | **FC** | **n** |
| Body weight (g) | 28.0 ± 0.7 | 40 | 27.9 ± 0.9 | 31 |
| Heart weight (mg) | 105.9 ± 3.1 | 40 | 105.5 ± 4.0 | 31 |
| Lung weight (mg) | 155.6 ± 4.0 | 35 | 148.2 ± 3.5 | 30 |
| Heart weight / Body Weight (mg/g) | 3.8 ± 0.1 | 40 | 3.8 ± 0.1 | 31 |
| Lung weight /Body weight (mg(g) | 5.6 ± 0.2 | 35 | 5.4 ± 0.2 | 30 |
| | | | | |

| **By sex** | **M** | **n** | **F** | **n** |
|---|---|---|---|---|
| Body weight (g) | 33.2 ± 1.2 | 19 | 26.0± 0.3 * | 52 |
| Heart weight (mg) | 127.9 ± 5.6 | 19 | 97.6 ± 1.6 $ | 52 |
| Lung weight (mg) | 165.6 ± 5.3 | 19 | 146.7 ± 2.8 # | 46 |
| Heart weight / Body Weight (mg/g) | 3.9 ± 0.1 | 19 | 3.8 ± 0.1 | 52 |
| Lung weight /Body weight (mg(g) | 5.1 ± 0.2 | 19 | 5.7 ± 0.1 & | 46 |
| | * p<0.0001, $ p<0.0001, # p=0.0009, & p=0.021 | | | |

| **Males by genotype** | **M FF** | **n** | **M FC** | **n** |
|---|---|---|---|---|
| Body weight (g) | 33.4 ± 1.7 | 10 | 32.9± 2.0 | 9 |
| Heart weight (mg) | 126.3 ± 8.4 | 10 | 129.7 ± 7.9 | 9 |
| Lung weight (mg) | 170.1 ± 10.9 | 8 | 161.8 ± 6.0 | 9 |
| Heart weight / Body Weight (mg/g) | 3.8 ± 0.2 | 10 | 4.0 ± 0.2 | 9 |
| Lung weight /Body weight (mg(g) | 5.2 ± 0.4 | 10 | 5.0 ± 0.2 | 9 |
| | | | | |

| **Females by genotype** | **F FF** | **n** | **F FC** | **n** |
|---|---|---|---|---|
| Body weight (g) | 26.2 ± 0.5 | 30 | 25.9 ± 0.5 | 22 |
| Heart weight (mg) | 99.1 ± 2.0 | 30 | 95.6 ± 2.5 | 22 |
| Lung weight (mg) | 150.2 ± 4.0 | 30 | 142.4 ± 3.6 | 21 |
| Heart weight / Body Weight (mg/g) | 3.8 ± 0.1 | 30 | 3.7 ± 0.1 | 22 |
| Lung weight /Body weight (mg(g) | 5.8 ± 0.2 | 25 | 5.5 ± 0.2 | 21 |

| | | | | |
|---|---|---|---|---|
| FF = Serca2^{flox/flox} M = males FC = Serca2^{wt/flox} MLC-2v^{wt/cre} F= females | | | | |

**Table 3**

| **Heart dimensions and flow parameters** | | | |
|---|---|---|---|
| Echocardiographic data | | | |
| | | FF | FC |
| | n | 14 | 14 |
| M-mode | | | |
| | IVSd (mm) | 0.97 ± 0.02 | 0.99 ± 0.02 |
| | IVSs (mm) | 1.56 ± 0.04 | 1.59 ± 0.04 |
| | IVS thickening (%) | 61 ± 3 | 61 ± 4 |
| | LVDd (mm) | 3.76 ± 0.08 | 3.60 ± 0.07 |
| | LVDs (mm) | 2.36 ± 0.10 | 2.16 ± 0.09 |
| | FS (%) | 37 ± 2 | 40 ± 2 |
| | PWd (mm) | 0.89 ± 0.02 | 0.88 ± 0.03 |
| | PWs (mm) | 1.34 ± 0.05 | 1.34 ± 0.06 |
| | PW thickening (%) | 52 ± 6 | 53 ± 5 |
| | PWSV (cm s⁻¹) | 2.12 ± 0.11 | 2.02 ± 0.08 |
| | PWRV (cm s⁻¹) | 2.28 ± 0.14 | 2.5 ± 0.13 |
| | | | |

| 2-D | | | |
|---|---|---|---|
| | IVSd (mm) | 1.01 ± 0.03 | 0.99 ± 0.03 |
| | IVSs (mm) | 1.42 ± 0.03 | 1.44 ± 0.02 |
| | IVS thickening (%) | 44 ± 3 | 44 ± 2 |
| | LVDd (mm) | 3.62 ± 0.12 | 3.50 ± 0.10 |
| | LVDs (mm) | 2.49 ± 0.11 | 2.37 ± 0.09 |
| | FS (%) | 31 ± 1 | 32 ± 1 |
| | PWd (mm) | 0.94 ± 0.02 | 0.93 ± 0.03 |
| | PWS (mm) | 1.34 ± 0.05 | 1.35 ± 0.04 |
| | PW thickening (%) | 45 ± 3 | 50 ± 3 |
| | Aorta (mm) | 1.38 ± 0.03 | 1.36 ± 0.02 |
| | | | |

| Doppler | | | |
|---|---|---|---|
| | Peak mitral flow (m s⁻¹) | 0.68 ± 0.03 | 0,72 ± 0,03 |
| | Peak LVOT flow (m s⁻¹) | 1.28 ± 0.06 | 1.24 ± 0,06 |
| | Peak RVOT flow (m s⁻¹) | 0.59 ± 0.03 | 0.54 ± 0.03 |
| | VTI in LVOT | 4.44 ± 0.21 | 4.23 ± 0,24 |
| | CO in LVOT (ml min⁻¹) | 31.5 ± 2.3 | 31.7 ± 2,1 |

| | | | |
|---|---|---|---|
| FF, Serca2^{flox/flox}; FC, Serca2^{wt/flox} MLC-2v^{wt/cre}; IVSd/s, inter-ventricular septum thickness in diastole respectively systole; LVDd/s, left ventricular diameter in diastole respectively systole; FS, fractional shortening in LVD; PWd/s, posterior wall thickness in diastole respectively systole; LAD, left atrial diameter; PWSV, posterior wall shortening velocity; PWRV, posterior wall relaxation velocity; LVOT, left ventricular outlet tract; RVOT, right ventricular outlet tract; CO, cardiac output; VTI, velocity time integral All values are mean ± SEM | | | |

**Table 4: Haemodynamic pressure measurements.**

| **Pressure data** | | |
|---|---|---|
| **Genotype** | **FF** | **FC** |
| n | 14 | 16 |
| | | |
| HR (beats/min) | 551 ± 25 | 538 ± 19 |
| LVSP (mm Hg) | 115,5 ± 5,0 | 111,6 ± 5,0 |
| LVEDP (mm Hg) | 5,0 ± 0,3 | 6,3 ± 1,5 |
| LVdP/dtmax | 9772 ± 811 | 8402 ± 749 |
| LVdP/dtmin | -10020 ± 681 | -8840 ± 700 |

| | | |
|---|---|---|
| Genotypes: (Control) FF = Serca2^{flox/flox} (Cardiac heterozygous) FC = Serca2^{wt/flox} MLC-2v^{wt/cre} | | |

**Table 5**

| **Reduction of functional cardiac Ca²⁺ ATPase protein content in test model.** Measurement of cardiac Ca²⁺ ATPase content in control and heterozygous animals (Mean ± SEM). | | | |
|---|---|---|---|
| **Age** | **nmol Ca²⁺ ATPase / g protein** | | **Reduction ATPAse** |
| (weeks) | **FF** | **FC** | **content (%)** |
| 11 | 47 (n=2) | 26 (n=2) | 32 |
| 19 | 32 ± 3 | 23 ± 1 * | 28 |
| | (n=7) | (n=5) | |

| | | | |
|---|---|---|---|
| * p<0.0005 FF = Serca2^{flox/flox} FC = Serca2^{wt/flox} MLC-2v^{wt/cre} | | | |

**Table 6 Breeding of tamoxifen-inducible cardiac Serca2 knockout mice**

| **Cross** | | **Total** | **wt** | **MCM** | **Serca2^{wt/flox}** | **Serca2^{wt/flox} MCM** | **Serca2^{flox/flox}** | **Serca2^{flox/flox} MCM** |
|---|---|---|---|---|---|---|---|---|
| Serca2^{wt/flox} X MCM | n | 38 | 7 | 9 | 12 | 10 | | |
| | % | | 18 | 24 | 32 | 26 | | |
| | | | | | | | | |
| Serca2^{flox/flox} X Serca2^{wt/flox} MCM | n | 19 | | | 3 | 9 | 4 | 3 |
| | % | | | | 16 | 47 | 21 | 16 |
| | | | | | | | | |
| Serca2^{flox/flox} X Serca2^{flox/flox} MCM | n | 121 | | | | | 57 | 67 |
| | % | | | | | | 46 | 54 |

## Claims

1. A genetically modified mouse having its gnomic Serca ATPase gene modified by inserted recombination sites of heterogenous origin, said modification being homozygous.

2. The mouse of claim 1, comprising several copies of the modified Serca ATPase gene.

3. The mouse of claim 1, wherein the Serca ATPase gene is a Serca2 ATPase gene.

4. The mouse of claim 1, wherein the heterogenous recombination sites are of non-mammalian origin.

5. The mouse of claim 4, wherein the recombination sites comprise loxP recombination sites.

6. The mouse of claim 1, further comprising a gene encoding a heterogenous recombinase.

7. The mouse of claim 6, wherein the heterogenous recombinase is of non-mammalian origin.

8. The mouse of claim 7, wherein the recombinase is a Cre recombinase.

9. The mouse of claim 6, wherein expression of the recombinase encoding gene is controlled by a regulatory nucleic acid sequence.

10. The mouse of claim 9, wherein the regulatory nucleic acid sequence is inducible.

11. The mouse of claim 10, wherein said regulatory nucleic acid sequence is inducible by tamoxifen.

12. The mouse of claim 6, wherein expression of the recombinase gene is tissue-specific.

13. The mouse of claim 12, wherein expression of the recombinase gene occurs in heart tissue.

14. A eukaryotic cell having its genomic Serca ATPase gene modified by inserted recombination sites of heterogenous origin, said modification being homozygous, wherein said eukaryotic cell is not a human embryonic stem cell.

15. The cell of claim 14 comprising several copies of the modified Serca ATPase gene.

16. The cell of claim 14, wherein the Serca ATPase gene is a Serca2 ATPase gene.

17. The cell of claim 14, wherein the heterogenous recombination sites are of non-mammalian origin.

18. The cell of claim 17, wherein the recombination sites comprise loxP recombination sites.

19. The cell of claim 14 further comprising a gene encoding a heterogenous recombinase.

20. The cell of claim 19, wherein the heterogenous recombinase is of non-mammalian origin.

21. The cell of claim 20, wherein the recombinase is a Cre recombinase.

22. The cell of claim 19, wherein expression of the recombinase encoding gene is controlled by a regulatory nucleic acid sequence.

23. The cell of claim 22, wherein the regulatory nucleic acid sequence is inducible.

24. The cell of claim 14, wherein the cell is of mammalian origin.

25. The cell of claim 24, wherein the cell is of non-human origin.

26. The cell of claim 25, wherein the cell is of rodent origin.

27. The cell of claim 26, wherein the cell is of mouse origin.

28. The cell of anyone of claims 14-27, wherein said cell is a non-human embryonic cell.

29. The cell of anyone of claims 14-28, wherein said cell is a cardiomyocyte.

30. A gene encoding a Serca ATPase modified by inserted recombination sites, wherein said recombination sites are heterogenous to said gene.

31. The gene of claim 30, wherein the Serca ATPase is a Serca2 ATPase

32. The gene of claim 30, wherein the heterogenous recombination sites are of non-mammalian origin.

33. The gene of claim 32, wherein the recombination sites comprise loxP recombination sites.

34. The gene of claims 30, wherein said gene is modified as set forth in SEQ ID 1-3.

35. A vector comprising the gene of claim 30.

36. The vector of claim 35, wherein the vector is based on pBluescript II KS.

37. A method for screening a compound or a mixture of compounds for activity against defective Ca²⁺ handling, comprising the following steps:
- inducing expression of the recombinase, and with that inactivation of the Serca ATPase gene, in the mouse according to anyone of claims 6-13 and
- administrating the compound or a mixture of compounds to said mouse before and/or after the induced inactivation of the Serca ATPase gene and
- detecting whether the induced defective Ca²⁺ handling is normalized by the administration of said compound or mixture of compounds.

38. The method of claim 37, wherein the Serca ATPase gene is a Serca2 ATPase gene.

39. The method of claim 37, wherein expression of the recombinase gene occurs in heart tissue.

40. The method of claim 39, wherein said method is suitable for screening a compound or a mixture of compounds for activity against heart failure.

## Patentansprüche

1. Genetisch modifizierte Maus, deren genomisches Serca-ATPase-Gen durch eingefügte Rekombinationsstellen heterogenen Ursprungs modifiziert ist, wobei die Modifikation homozygot ist.

2. Maus nach Anspruch 1, mehrere Kopien des modifizierten Serca-ATPase-Gens enthaltend.

3. Maus nach Anspruch 1, wobei das Serca-ATPase-Gen ein Serca2-ATPase-Gen ist.

4. Maus nach Anspruch 1, wobei die heterogenen Rekombinationsstellen nicht von einem Säugetier stammen.

5. Maus nach Anspruch 4, wobei die Rekombinationsstellen loxP-Rekombinationsstellen beinhalten.

6. Maus nach Anspruch 1, weiter ein Gen enthaltend, das eine heterogene Rekombinase codiert.

7. Maus nach Anspruch 6, wobei die heterogene Rekombinase nicht von einem Säugetier stammt.

8. Maus nach Anspruch 7, wobei die Rekombinase eine Cre-Rekombinase ist.

9. Maus nach Anspruch 6, wobei die Expression des die Rekombinase codierenden Gens von einer regulatorischen Nukleinsäuresequenz gesteuert wird.

10. Maus nach Anspruch 9, wobei die regulatorische Nukleinsäuresequenz induzierbar ist.

11. Maus nach Anspruch 10, wobei die regulatorische Nukleinsäuresequenz durch Tamoxifen induzierbar ist.

12. Maus nach Anspruch 6, wobei die Expression des Rekombinase-Gens gewebespezifisch ist.

13. Maus nach Anspruch 12, wobei die Expression des Rekombinase-Gens in Herzgewebe erfolgt.

14. Eukaryotische Zelle, deren genomisches Serca-ATPase-Gen durch eingefügte Rekombinationsstellen heterogenen Ursprungs modifiziert ist, wobei die Modifikation homozygot ist und die eukaryotische Zelle keine menschliche embryonale Stammzelle ist.

15. Zelle nach Anspruch 14, mehrere Kopien des modifizierten Serca-ATPase-Gens enthaltend.

16. Zelle nach Anspruch 14, wobei das Serca-ATPase-Gen ein Serca2-ATPase-Gen ist.

17. Zelle nach Anspruch 14, wobei die heterogenen Rekombinationsstellen nicht von einem Säugetier stammen.

18. Zelle nach Anspruch 17, wobei die Rekombinationsstellen loxP-Rekombinationsstellen beinhalten.

19. Zelle nach Anspruch 14, weiter ein Gen enthaltend, das eine heterogene Rekombinase codiert.

20. Zelle nach Anspruch 19, wobei die heterogene Rekombinase nicht von einem Säugetier stammt.

21. Zelle nach Anspruch 20, wobei die Rekombinase eine Cre-Rekombinase ist.

22. Zelle nach Anspruch 19, wobei die Expression des die Rekombinase codierenden Gens von einer regulatorischen Nukleinsäuresequenz gesteuert wird.

23. Zelle nach Anspruch 22, wobei die regulatorische Nukleinsäuresequenz induzierbar ist.

24. Zelle nach Anspruch 14, wobei die Zelle von einem Säugetier stammt.

25. Zelle nach Anspruch 24, wobei die Zelle nicht menschlichen Ursprungs ist.

26. Zelle nach Anspruch 25, wobei die Zelle von einem Nagetier stammt.

27. Zelle nach Anspruch 26, wobei die Zelle von einer Maus stammt.

28. Zelle nach einem der Ansprüche 14 bis 27, wobei die Zelle eine nicht menschliche embryonale Zelle ist.

29. Zelle nach einem der Ansprüche 14 bis 28, wobei die Zelle ein Kardiomyozyt ist.

30. Gen, das eine durch eingefügte Rekombinationsstellen modifizierte Serca-ATPase codiert, wobei die Rekombinationsstellen in Bezug auf das Gen heterogen sind.

31. Gen nach Anspruch 30, wobei die Serca-ATPase eine Serca2-ATPase ist.

32. Gen nach Anspruch 30, wobei die heterogenen Rekombinationsstellen nicht von einem Säugetier stammen.

33. Gen nach Anspruch 32, wobei die Rekombinationsstellen loxP-Rekombinationsstellen beinhalten.

34. Gen nach Anspruch 30, wobei das Gen gemäss SEQ ID 1-3 modifiziert ist.

35. Vektor, der das Gen nach Anspruch 30 enthält.

36. Vektor nach Anspruch 35, wobei der Vektor auf pBluescript II KS basiert.

37. Verfahren zur Überprüfung einer Verbindung oder eines Gemischs von Verbindungen auf deren Wirksamkeit gegen eine gestörte Ca²⁺-Homöostase, mit folgenden Schritten:
- die Expression der Rekombinase induzieren und damit das Serca-ATPase-Gen in der Maus nach einem der Ansprüche 6 bis 13 inaktivieren und
- der Maus vor und/oder nach der induzierten Inaktivierung des Serca-ATPase-Gens die Verbindung oder ein Gemisch von Verbindungen verabreichen und
- feststellen, ob die induzierte gestörte Ca²⁺-Homöostase durch die Verabreichung der genannten Verbindung oder des genannten Gemischs von Verbindungen normalisiert wird.

38. Verfahren nach Anspruch 37, wobei das Serca-ATPase-Gen ein Serca2-ATPase-Gen ist.

39. Verfahren nach Anspruch 37, wobei die Expression des Rekombinase-Gens in Herzgewebe erfolgt.

40. Verfahren nach Anspruch 39, wobei sich das Verfahren zur Überprüfung einer Verbindung oder eines Gemischs von Verbindungen auf deren Wirksamkeit gegen Herzinsuffizienz eignet.

## Revendications

1. Souris génétiquement modifiée dont le gène génomique de la Serca-ATPase est modifié par l'insertion de sites de recombinaison d'origine hétérogène, ladite modification étant homozygote.

2. Souris selon la revendication 1, comprenant plusieurs copies du gène de la Serca-ATPase modifié.

3. Souris selon la revendication 1, où le gène de la Serca-ATPase est un gène de la Serca2-ATPase.

4. Souris selon la revendication 1, où les sites de recombinaison hétérogènes sont d'origine non mammifère.

5. Souris selon la revendication 4, où les sites de recombinaison comprennent des sites de recombinaison loxP.

6. Souris selon la revendication 1, comprenant en outre un gène codant une recombinase hétérogène.

7. Souris selon la revendication 6, où la recombinase hétérogène est d'origine non mammifère.

8. Souris selon la revendication 7, où la recombinase est une cre recombinase.

9. Souris selon la revendication 6, où l'expression du gène codant la recombinase est contrôlée par une séquence d'acide nucléique régulatrice.

10. Souris selon la revendication 9, où la séquence d'acide nucléique régulatrice est inductible.

11. Souris selon la revendication 10, où ladite séquence d'acide nucléique régulatrice est inductible par le tamoxifène.

12. Souris selon la revendication 6, où l'expression du gène de la recombinase est spécifique au tissu.

13. Souris selon la revendication 12, où l'expression du gène de la recombinase a lieu dans le tissu cardiaque.

14. Cellule eucaryote dont le gène génomique de la Serca-ATPase est modifié par l'insertion de sites de recombinaison d'origine hétérogène, ladite modification étant homozygote, où ladite cellule eucaryote n'est pas une cellule souche embryonnaire humaine.

15. Cellule selon la revendication 14, comprenant plusieurs copies du gène de la Serca-ATPase modifié.

16. Cellule selon la revendication 14, où le gène de la Serca-ATPase est un gène de la Serca2-ATPase.

17. Cellule selon la revendication 14, où les sites de recombinaison hétérogènes sont d'origine non mammifère.

18. Cellule selon la revendication 17, où les sites de recombinaison comprennent des sites de recombinaison loxP.

19. Cellule selon la revendication 14, comprenant en outre un gène codant une recombinase hétérogène.

20. Cellule selon la revendication 19, où la recombinase hétérogène est d'origine non mammifère.

21. Cellule selon la revendication 20, où la recombinase est une cre recombinase.

22. Cellule selon la revendication 19, où l'expression du gène codant la recombinase est contrôlée par une séquence d'acide nucléique régulatrice.

23. Cellule selon la revendication 22, où la séquence d'acide nucléique régulatrice est inductible.

24. Cellule selon la revendication 14, où la cellule est d'origine mammifère.

25. Cellule selon la revendication 24, où la cellule est d'origine non humaine.

26. Cellule selon la revendication 25, où la cellule est issue d'un rongeur.

27. Cellule selon la revendication 26, où la cellule est issue d'une souris.

28. Cellule selon l'une quelconque des revendications 14 à 27, où ladite cellule est une cellule embryonnaire non humaine.

29. Cellule selon l'une quelconque des revendications 14 à 28, où ladite cellule est un cardiomyocyte.

30. Gène codant une Serca-ATPase modifiée par l'insertion de sites de recombinaison, où lesdits sites de recombinaison sont hétérogènes audit gène.

31. Gène selon la revendication 30, où la Serca-ATPase est une Serca2-ATPase.

32. Gène selon la revendication 30, où les sites de recombinaison hétérogènes sont d'origine non mammifère.

33. Gène selon la revendication 32, où les sites de recombinaison comprennent des sites de recombinaison loxP.

34. Gène selon la revendication 30, où ledit gène est modifié selon la SEQ ID 1-3.

35. Vecteur comprenant le gène selon la revendication 30.

36. Vecteur selon la revendication 35, où le vecteur est basé sur pBluescript II KS.

37. Procédé pour l'examen d'un composé ou un mélange de composés quant à son activité contre une déficience du métabolisme Ca2+, comprenant les étapes suivantes:
- induire l'expression de la recombinase, et avec celle-ci l'inactivation du gène de la Serca-ATPase dans la souris selon l'une quelconque des revendications 6 à 13 et
- administrer le composé ou un mélange de composés à ladite souris avant et/ou après l'inactivation induite du gène de la Serca-ATPase et
- détecter si la déficience du métabolisme Ca²⁺ induite est normalisée par l'administration dudit composé ou mélange de composés.

38. Procédé selon la revendication 37, où le gène de la Serca-ATPase est un gène de la Serca2-ATPase.

39. Procédé selon la revendication 37, où l'expression du gène de la recombinase a lieu dans le tissu cardiaque.

40. Procédé selon la revendication 39, où ledit procédé convient pour examiner un composé ou un mélange de composés quant à son activité contre l'insuffisance cardiaque.
